(19) Europäisches Patentamt
European Patent Office
Office européen des brevets

(11) Veröffentlichungsnummer: **0 183 650**
**B1**

(12) **EUROPÄISCHE PATENTSCHRIFT**

(45) Veröffentlichungstag der Patentschrift: **20.04.88**

(21) Anmeldenummer: **85810557.0**

(22) Anmeldetag: **21.11.85**

(51) Int. Cl.⁴: **C 07 C 109/06**, C 07 C 147/02, C 07 C 147/14, C 07 C 149/14, C 07 D 271/10, C 07 D 307/04, C 07 D 309/08, C 07 D 413/12, A 01 N 37/46, A 01 N 43/08, A 01 N 43/16

# (54) Phenylhydrazine, Verfahren zu ihrer Herstellung und ihre Verwendung in der Schädlingsbekämpfung.

(30) Priorität: **27.11.84 CH 5655/84**
**27.11.84 CH 5656/84**
**18.07.85 CH 3122/85**
**25.10.85 CH 4608/85**

(43) Veröffentlichungstag der Anmeldung: **04.06.86 Patentblatt 86/23**

(45) Bekanntmachung des Hinweises auf die Patenterteilung: **20.04.88 Patentblatt 88/16**

(84) Benannte Vertragsstaaten: **BE CH DE FR GB IT LI NL**

(56) Entgegenhaltungen:
**EP - A - 0 000 002**
**DE - A - 3 047 629**
**FR - B - 2 466 194**
**GB - A - 2 093 448**

(73) Patentinhaber: **CIBA-GEIGY AG, Klybeckstrasse 141, CH-4002 Basel (CH)**

(72) Erfinder: **Ehrenfreund, Josef, Dr., Amselstrasse 11, CH-4123 Allschwil (CH)**

EP 0 183 650 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Phenylhydrazine, Verfahren zu ihrer Herstellung und ihre Verwendung in der Schädlingsbekämpfung.

Die Phenylhydrazine haben die Formel I

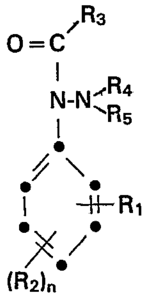

(I)

worin

$R_1$ -A-R-Z,

A Sauerstoff, Schwefel, -S- oder -S- ,
O O
|| ||
||
O

R $C_1$-$C_6$-Alkylen, $C_1$-$C_6$-Halogenalkyl-$C_1$-$C_6$-alkylen, $C_1$-$C_6$-Alkoxy-$C_1$-$C_6$-alkylen oder $C_1$-$C_6$-Alkylthio-$C_1$-$C_6$-alkylen,

Z Halogen, $C_1$-$C_6$-Halogenalkyl, $C_1$-$C_6$-Alkoxy, $C_1$-$C_6$-Alkylthio, $C_1$-$C_6$-Alkylsulfinyl oder $C_1$-$C_6$-Alkylsulfonyl, oder

R und Z zusammen 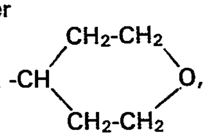,

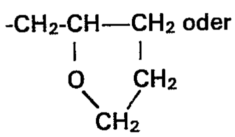 oder 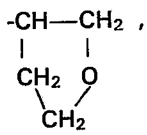 ,

$R_2$ Wasserstoff, Halogen, $C_1$-$C_6$-Alkyl, $C_1$-$C_6$-Alkoxy, $C_1$-$C_6$-Alkylthio oder ($C_1$-$C_6$-Alkyl)$_2$amino,

n die Zahlen 1-4, wobei $R_2$ verschieden sein kann, falls n eine der Zahlen 2-4 ist,

$R_3$ Halogen,

$R_4$ Wasserstoff,

$R_5$ -$COOR_6$ oder

$R_3$, $R_4$ und $R_5$ zusammen ein Brückenglied der Formel -O-C-$OR_6$
||
O

und

$R_6$ $C_1$-$C_6$-Alkyl, $C_2$-$C_6$-Alkenyl oder $C_2$-$C_6$-Alkinyl bedeuten.

Unter Halogen bei R, $R_2$, $R_3$ und Z ist Fluor, Chlor, Brom oder Jod zu verstehen. Bei $R_3$ kommt insbesondere Fluor oder Chlor, aber ganz besonders Fluor, in Frage.

Die Alkyl-, Alkoxy-, Alkylthio-, Alkylsulfinyl-, Alkylsulfonyl-, Alkenyl- und Alkinylgruppen bei R, $R_2$, $R_6$ und Z können geradkettig oder verzweigt und gegebenenfalls durch Halogen, $C_1$-$C_6$-Alkoxy oder $C_1$-$C_6$-Alkylthio substituiert sein. Beispiele solcher Gruppen sind u.a.: Methyl, Methoxy, Methylthio, Trifluormethyl, Äthyl, Äthoxy, Pentafluoralkoxy, Propyl, Propoxy, Propylthio, Isopropyl, Isopropoxy, Isopropylthio, n-Butyl, n-Butoxy, n-Butylthio, n-Pentyl, n-Pentoxy, n-Pentylthio, n-Hexyl, n-Hexoxy, n-Hexylthio und deren Isomere, 2-Methylthioäthoxy, 2-Methoxyäthoxy, Propenyl oder Propinyl.

Die Alkylengruppen bei R können geradkettig oder verzweigt sein. Beispiele solcher Gruppen sind u.a.:

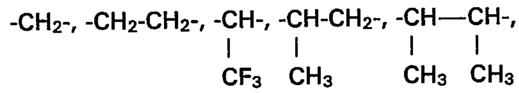

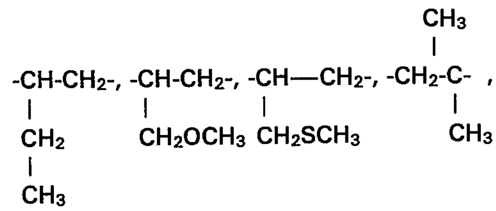

$-CH_2-CH_2-CH_2-$, $-CH_2-(CH_2)_2-CH_2-$, $-CH_2-(CH_2)_3-CH_2-$, $-CH_2-(CH_2)_4-CH_2-$.

Die Verbindungen der Formel I sind offenkettige Phenylhydrazine der Formel

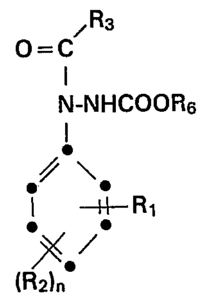

(Ia)

worin $R_1$, $R_2$, $R_3$, $R_6$ und n die für die Formel I angegebene Bedeutung haben oder Oxadiazolinone der Formel

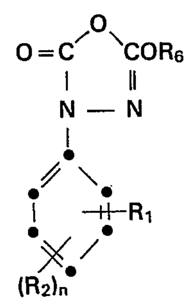

(Ib)

worin $R_1$; $R_2$, $R_6$ und n die für die Formel I angegebene Bedeutung haben.

Bevorzugt sind Verbindungen der Formel Ia worin

$R_1$ -A-R-Z,

A Sauerstoff, Schwefel, -S- oder -S- ,
O
||
|| ||
O O

R $C_2$-$C_6$-Alkylen,

Z Halogen, $C_1$-$C_6$-Halogenalkyl, $C_1$-$C_6$-Alkoxy, $C_1$-$C_6$-Alkylthio, $C_1$-$C_6$-Alkylsulfinyl oder $C_1$-$C_6$-Alkylsulfonyl, oder

R und Z zusammen

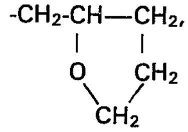, 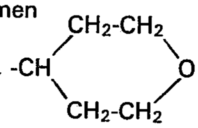 oder

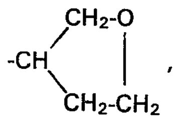 ,

$R_2$ Wasserstoff, Halogen, $C_1$-$C_6$-Alkyl, $C_1$-$C_6$-Alkoxy, $C_1$-$C_6$-Alkylthio oder ($C_1$-$C_6$-Alkyl)$_2$amino,
n die Zahlen 1-4, wobei $R_2$ verschieden sein kann, falls n eine der Zahlen 2-4 ist,
$R_3$ Halogen und
$R_6$ $C_1$-$C_6$-Alkyl, $C_2$-$C_6$-Alkenyl oder $C_2$-$C_6$-Alkinyl bedeuten oder

Verbindungen der Formel Ib, worin

$R_1$ -A-R-Z,
A Sauerstoff, Schwefel, $-\underset{O}{\underset{\|}{S}}-$ oder $-\overset{O}{\overset{\|}{\underset{O}{\underset{\|}{S}}}}-$ ,
R $C_2$-$C_6$-Alkylen,
Z Halogen, $C_1$-$C_6$-Halogenalkyl, $C_1$-$C_6$-Alkoxy, $C_1$-$C_6$-Alkylthio, $C_1$-$C_6$-Alkylsulfinyl oder $C_1$-$C_6$-Alkylsulfonyl, oder

R und Z zusammen

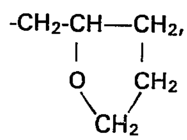, 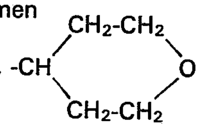 oder

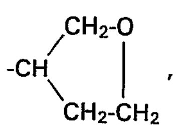 ,

$R_2$ Wasserstoff, Halogen, $C_1$-$C_6$-Alkyl, $C_1$-$C_6$-Alkoxy, $C_1$-$C_6$-Alkylthio oder ($C_1$-$C_6$-Alkyl)$_2$amino,
n die Zahlen 1-4, wobei $R_2$ verschieden sein kann, falls n eine der Zahlen 2-4 ist, und
$R_6$ $C_1$-$C_6$-Alkyl, $C_2$-$C_6$-Alkenyl oder $C_2$-$C_6$-Alkinyl bedeuten oder

Verbindungen der Formel Ib, worin

$R_1$ -A-R-Z,
A Sauerstoff, Schwefel, $-\underset{O}{\underset{\|}{S}}-$ oder $-\overset{O}{\overset{\|}{\underset{O}{\underset{\|}{S}}}}-$ ,
R $C_2$-$C_6$-Alkylen,
Z Halogen, $C_1$-$C_6$-Alkoxy, $C_1$-$C_6$-Alkylthio, $C_1$-$C_6$-Alkylsulfinyl oder $C_1$-$C_6$-Alkylsulfonyl,
$R_2$ Wasserstoff, Halogen, $C_1$-$C_6$-Alkoxy, $C_1$-$C_6$-Alkylthio oder ($C_1$-$C_6$-Alkyl)$_2$amino,
n die Zahlen 1-4, wobei $R_2$ verschieden sein kann, falls n eine der Zahlen 2-4 ist, und
$R_6$ $C_1$-$C_6$-Alkyl, $C_2$-$C_6$-Alkenyl oder $C_2$-$C_6$-Alkinyl bedeuten.

Insbesondere bevorzugt sind Verbindungen der Formel Ia, worin
$R_1$ -A-R-Z,
A Sauerstoff oder Schwefel,
$R_1$ unsubstituiertes $C_2$-$C_6$-Alkylen,
Z Halogen, $C_1$-$C_6$-Alkoxy, $C_1$-$C_6$-Alkylthio, $C_1$-$C_6$-Alkylsulfinyl oder $C_1$-$C_6$-Alkylsulfonyl,
$R_2$ Wasserstoff, Halogen oder $C_1$-$C_6$-Alkyl,
n die Zahl 1,
$R_3$ Halogen und
$R_6$ Methyl bedeuten oder Verbindungen der Formel Ia, worin
$R_1$ -A-R-Z in 2-Position zur

Gruppe 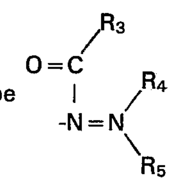 steht,

A Sauerstoff,
R unsubstituiertes $C_2$-$C_4$-Alkylen,
Z Halogen, $C_1$-$C_4$-Alkoxy, $C_1$-$C_4$-Alkylthio, $C_1$-$C_4$-Alkylsulfinyl oder $C_1$-$C_4$-Alkylsulfonyl,
$R_2$ Wasserstoff, Halogen oder $C_1$-$C_4$-Alkyl,
n die Zahl 1,
$R_3$ Halogen und
$R_6$ Methyl bedeuten oder Verbindungen der Formel Ib, worin
$R_1$ -A-R-Z,
A Sauerstoff oder Schwefel,
R $C_2$-$C_6$-Alkylen,
Z Halogen, $C_1$-$C_6$-Alkoxy, $C_1$-$C_6$-Alkylthio, $C_1$-$C_6$-Alkylsulfinyl oder $C_1$-$C_6$-Alkylsulfonyl,
$R_2$ Wasserstoff, Halogen oder $C_1$-$C_6$-Alkyl,
n die Zahl 1 und
$R_6$ Methyl bedeuten oder Verbindungen der Formel Ib, worin
$R_1$ -A-R-Z in 2-Position zur

Gruppe 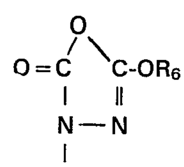 steht,

A Sauerstoff,
R unsubstituiertes $C_2$-$C_4$-Alkylen,
Z Halogen, $C_1$-$C_4$-Alkoxy, $C_1$-$C_4$-Alkylthio, $C_1$-$C_4$-Alkylsulfinyl oder $C_1$-$C_4$-Alkylsulfonyl,
$R_2$ Wasserstoff, Halogen oder $C_1$-$C_4$-Alkyl,
n die Zahl 1
und
$R_6$ Methyl bedeuten.

Beispiele von Verbindungen der Formel I sind u.a.

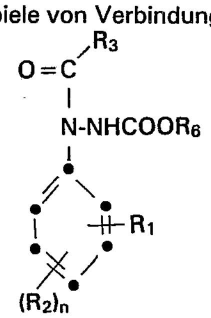

| $R_3$ | $R_1$ | $R_2$ | n | $R_6$ |
|---|---|---|---|---|
| Cl | $2\text{-}O(CH_2)_3SCH_3$ | H | 1 | $CH_3$ |
| F | $2\text{-}OCH_2CHOCH_3$<br>$CH_3$ | H | 1 | $C_2H_5$ |
| F | $2\text{-}OCH_2CF_3$ | H | 1 | $CH_3$ |
| F | $2\text{-}OCHCH_2OCH_3$<br>$CH_3$ | $6\text{-}CH_3$ | 1 | $n\text{-}C_4H_9$ |
| F | $2\text{-}O(CH_2)_2SCH_3$ | $5\text{-}CH_3$ | 1 | $-CH_2CH=CH_2$ |
| Cl | $2\text{-}O(CH_2)_2SCH_3$ | $3,5\text{-}CH_3$ | 2 | $-CH_2C\equiv CH$ |
| F | $2\text{-}O(CH_2)_2SCH_3$ | $3,5\text{-}CH_3$ | 2 | $CH_3$ |
| F | $2\text{-}O(CH_2)_2SCH_3$ | H | 1 | $n\text{-}C_6H_{13}$ |
| F | $2\text{-}O(CH_2)_2SCH_3$ | H | 1 | $CH_3$ |
| Cl | $2\text{-}O(CH_2)_2SCH_3$ | $4\text{-}CH_3$ | 1 | $CH_3$ |
| F | $2\text{-}O(CH_2)_2SCH_3$ | $4\text{-}CH_3$ | 1 | $C_2H_5$ |
| F | $2\text{-}OCH_2CHOCH_3$<br>$CF_3$ | $5\text{-}CH_3$ | 1 | $n\text{-}C_4H_9$ |
| F | $2\text{-}OCH_2CHOCH_3$<br>$CH_3$ | $3,5\text{-}CH_3$ | 2 | $n\text{-}C_4H_9$ |
| F | $2\text{-}OCH_2CHOCH_3$<br>$CH_3$ | $4,5\text{-}CH_3$ | 2 | $CH_3$ |
| F | $2\text{-}OCHCH_2OCH_3$<br>$CH_3$ | $4\text{-}CH_3$ | 1 | $CH_3$ |
| F | $2\text{-}OCHCH_2OCH_3$<br>$CH_3$ | $3\text{-}CH_3$ | 1 | $CH_3$ |
| Cl | $2\text{-}OCHCH_2OCH_3$<br>$CH_3$ | $3,4,5\text{-}CH_3$ | 3 | $CH_3$ |
| Cl | $2\text{-}O(CH_2)_2OCH_3$ | $3,4,5\text{-}CH_3$ | 3 | $CH_3$ |
| Cl | $2\text{-}OCH_2\text{-}CH\text{-}OCH_3$<br>$CH_3$ | $3,4,5\text{-}CH_3$ | 3 | $n\text{-}C_4H_9$ |
| F | $2\text{-}OCHCH_2OCH_3$<br>$CH_3$ | $4,5\text{-}CH_3$ | 2 | $-CH_2\text{-}CH_2=CH_2$ |
| Cl | $2\text{-}OCHCH_2OCH_3$<br>$CH_3$ | $5\text{-}C_3H_{7(i)}$ | 1 | $CH_3$ |
| F | $2\text{-}OCHCH_2OCH_3$<br>$CH_3$ | $5\text{-}C_3H_{7(i)}$ | 1 | $CH_3$ |

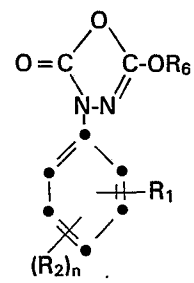

| $R_1$ | $R_2$ | n | $R_6$ |
|---|---|---|---|
| 2-O$(CH_2)_2SCH_3$ | 4-$CH_3$ | 1 | $CH_3$ |
| 2-O$(CH_2)_2SCH_3$ | 3,5-$CH_3$ | 2 | $C_2H_5$ |
| 2-O$(CH_2)_2SO_2CH_3$ | 3,5-$CH_3$ | 2 | $CH_3$ |
| 2-O$(CH_2)_2SO_2CH_3$ | 4-$CH_3$ | 1 | $CH_3$ |
| 2-O$(CH_2)_2SO_2CH_3$ | 3-$CH_3$ | 1 | $CH_3$ |
| 2-O$(CH_2)_2OCH_3$ | 4-$CH_3$ | 1 | n-$C_4H_9$ |
| 2-O$(CH_2)_2OCH_3$ | 3-$CH_3$ | 1 | $CH_2$-$CH=CH_2$ |
| 2-O$(CH_2)_2OCH_3$ | 6-$CH_3$ | 1 | $CH_3$ |
| 2-O$(CH_2)_2OCH_3$ | 3,4-$CH_3$ | 2 | $CH_3$ |
| 2-O$(CH_2)_2OCH_3$ | 3,5-$CH_3$ | 2 | $CH_3$ |
| 2-O$(CH_2)_2OCH_3$ | 4,5-$CH_3$ | 2 | $CH_3$ |
| 2-O$(CH_2)_2OCH_3$ | 4,6-$CH_3$ | 2 | $CH_3$ |
| 2-O$CHCH_2OCH_3$<br>$CH_3$ | 3,4-$CH_3$ | 2 | $CH_3$ |
| 2-O$CHCH_2OCH_3$<br>$CH_3$ | 4,5-$CH_3$ | 2 | $CH_3$ |
| 2-O$CHCH_2OCH_3$<br>$CH_3$ | 4,6-$CH_3$ | 2 | $CH_3$ |
| 2-O$CHCH_2OCH_3$<br>$CH_3$ | 3-$CH_3$ | 1 | $C_2H_5$ |
| 2-O$CHCH_2OCH_3$<br>$CH_3$ | 4-$CH_3$ | 1 | n-$C_4H_9$ |
| 2-O$(CH_2)_2SCH_3$ | 3-$CH_3$ | 1 | n-$C_6H_{13}$ |
| 2-O$CHCH_2OCH_3$<br>$CH_3$ | 3,4,5-$CH_3$ | 3 | $CH_3$ |
| 2-O$(CH_2)_2OCH_3$ | 3,4-$CH_3$ | 2 | $CH_3$ |
| 2-O$(CH_2)_2SCH_3$ | 3,4-$CH_3$ | 2 | $CH_3$ |
| 2-O$(CH_2)_2SCH_3$ | 4,5-$CH_3$ | 2 | $CH_3$ |
| 2-O$(CH_2)_2SCH_3$ | 4,6-$CH_3$ | 2 | $CH_3$ |
| 2-O$(CH_2)_2SO_2CH_3$ | 3,4-$CH_3$ | 2 | $CH_3$ |
| 2-O$(CH_2)_2SO_2CH_3$ | 4,5-$CH_3$ | 2 | $CH_3$ |
| 2-O$(CH_2)_2SO_2CH_3$ | 4,6-$CH_3$ | 2 | $CH_3$ |
| 2-O$(CH_2)_2SO_2CH_3$ | 6-$CH_3$ | 1 | $CH_2$-$C\equiv CH$ |

<table>
<tr><th>$R_1$</th><th>$R_2$</th><th>n</th><th>$R_6$</th></tr>
<tr><td>2-O$(CH_2)_2SCH_3$</td><td>6-$CH_3$</td><td>1</td><td>$CH_2$-CH=$CH_2$</td></tr>
<tr><td>2-$OCH_2CHOCH_3$<br>|<br>$CF_3$</td><td>5-$CH_3$</td><td>1</td><td>n-$C_4H_9$</td></tr>
<tr><td>2-$OCH_2CHOCH_3$<br>|<br>$CH_3$</td><td>4-$CH_3$</td><td>1</td><td>n-$C_3H_7$</td></tr>
<tr><td>2-$OCH_2CHOCH_3$<br>|<br>$CH_3$</td><td>5-$CH_3$</td><td>1</td><td>n-$C_3H_7$</td></tr>
<tr><td>2-$OCH_2CHOCH_3$<br>|<br>$CH_3$</td><td>6-$CH_3$</td><td>1</td><td>n-$C_3H_7$</td></tr>
<tr><td>2-$OCH_2CHOCH_3$<br>|<br>$CH_3$</td><td>3,4-$CH_3$</td><td>2</td><td>i-$C_3H_7$</td></tr>
<tr><td>2-$OCH_2CHOCH_3$<br>|<br>$CH_3$</td><td>3,5-$CH_3$</td><td>2</td><td>i-$C_3H_7$</td></tr>
<tr><td>2-$OCH_2CHOCH_3$<br>|<br>$CH_3$</td><td>4,5-$CH_3$</td><td>2</td><td>i-$C_3H_7$</td></tr>
<tr><td>2-$OCH_2CHOCH_3$<br>|<br>$CH_3$</td><td>4,6-$CH_3$</td><td>2</td><td>i-$C_3H_7$</td></tr>
<tr><td>2-$OCH_2CHOCH_3$<br>|<br>$CH_3$</td><td>3,4-$CH_3$</td><td>2</td><td>$CH_3$</td></tr>
<tr><td>2-O$(CH_2)_3OCH_3$</td><td>H</td><td>1</td><td>$CH_3$</td></tr>
<tr><td>2-O$(CH_2)_3SCH_3$</td><td>H</td><td>1</td><td>n-$C_4H_9$</td></tr>
<tr><td>$CH_3$<br>|<br>2-$OCCH_2OCH_3$<br>|<br>$CH_3$</td><td>H</td><td>1</td><td>$CH_3$</td></tr>
<tr><td>$CH_3$<br>|<br>2-$OCH_2COCH_3$<br>|<br>$CH_3$</td><td>H</td><td>1</td><td>$C_2H_5$</td></tr>
<tr><td>2-OCH-$CHOCH_3$<br>| |<br>$CH_3CH_3$</td><td>H</td><td>1</td><td>$CH_3$</td></tr>
<tr><td>2-OCH-$CHOCH_3$<br>| |<br>$CH_3CH_3$</td><td>H</td><td>1</td><td>$C_2H_5$</td></tr>
<tr><td>2-OCH-$CHOCH_3$<br>| |<br>$CH_3CH_3$</td><td>5-$CH_3$</td><td>1</td><td>n-$C_3H_7$</td></tr>
<tr><td>2-$OCH_2CHSCH_3$<br>|<br>$CH_3$</td><td>H</td><td>1</td><td>i-$C_3H_7$</td></tr>
</table>

| $R_1$ | $R_2$ | n | $R_6$ |
|---|---|---|---|
| $CH_3$<br>$2\text{-}OCH_2CHOCH_3$<br>$CH_3$ | H | 1 | $i\text{-}C_3H_7$ |
| $2\text{-}O(CH_2)_3SO_2CH_3$ | H | 1 | $-CH_2-CH=CH_2$ |
| $2\text{-}OCHCH_2OCH_3$<br>$CH_3$ | $5\text{-}C_2H_5$ | 1 | $CH_3$ |
| $2\text{-}OCHCH_2OCH_3$<br>$CH_3$ | $5\text{-}C_3H_{7(i)}$ | 1 | $CH_3$ |
| $2\text{-}OCHCH_2OCH_3$<br>$CH_3$ | $5\text{-}C_4H_{9(t)}$ | 1 | $CH_3$ |
| $2\text{-}OCH_2CF_3$ | $5\text{-}CH_3$ | 2 | $CH_3$ |

Die Verbindungen der Formel I können nach an sich bekannten Methoden hergestellt werden, indem man in einer ersten Stufe durch Phosgenierung oder Fluorphosgenierung Verbindungen der Formel I herstellt, worin $R_5 = -COOR_6$ bedeutet und diese offenkettigen Hydrazine anschliessend mittels einer Base zu Verbindungen der Formel I, worin $R_3$, $R_4$ und $R_5$ zusammen $-O-C=$ bedeuten, cyclisiert:
$OR_6$

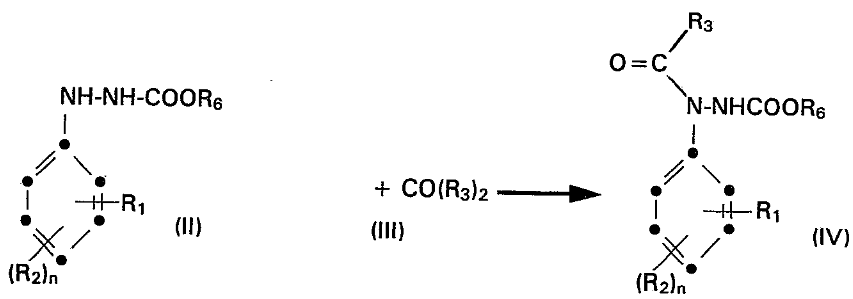

(II) (III) (IV)

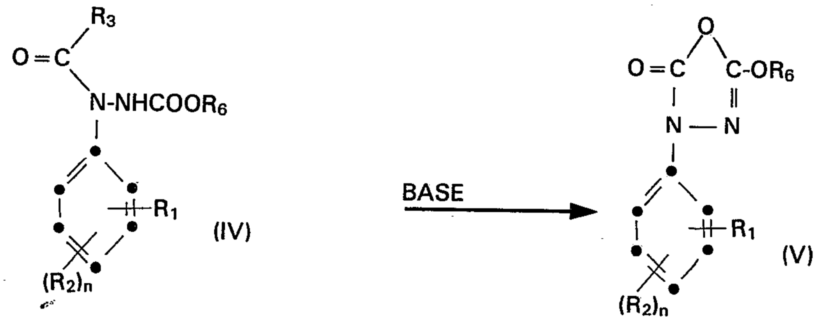

(IV) (V)

In den Formeln II-V haben $R_1$, $R_2$, $R_3$, $R_6$ und n die für die Formel I angegebene Bedeutung.

Als Basen für den zweiten Verfahrensschritt sind insbesondere organische Basen, wie Trialkylamine, z.B. Triäthylamin oder Äthyldiisopropylamin, Pyridin, Dialkylaniline oder cyclische Amidinbasen geeignet.

Beide Verfahrensschritte werden bei einer Reaktionstemperatur zwischen –10°C und 120°C, meist zwischen 20° und 80°C, bei normalem oder erhöhtem Druck und vorzugsweise in einem inerten Lösungs- oder Verdünnungsmittel durchgeführt. Als Lösungs- oder Verdünnungsmittel eignen sich z.B. Äther und ätherartige Verbindungen, wie Diäthyläther, Dipropyläther, Dioxan, Dimethoxyäthan und Tetrahydrofuran; Amide, wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon sowie Alkohole (nur beim Cyclisierungsschritt), z.B. Methanol, Äthanol, Propylalkohol usw. und Ester, z.B. Essigsäureäthylester.

Die Ausgangsstoffe der Formeln II können analog bekannten Methoden hergestellt werden (vgl. Beispiel 1).

Die Verbindungen der Formel I, welche S in seinen oxidierten Formen, also als

$$\underset{\underset{O}{\|}}{-S-} \quad \text{oder} \quad \overset{\overset{O}{\|}}{\underset{\underset{O}{\|}}{-S-}}$$

besitzen, können auch durch Oxidation der entsprechenden Verbindungen I, in welchen der Schwefel als Sulfid vorliegt, erhalten werden. Solche Oxidationen sind in der Literatur wohlbekannt und werden z.B. mittels Wasserstoffperoxid, Alkaliperjodaten oder organischen Persäuren wie Perbenzoesäure, 3-Chlorperbenzoesäure oder Peressigsäure durchgeführt. Weitere für solche Reaktionen geeignete Reagenzien sind in der Monographie L.F. Fieser und M. Fieser, Reagents for Organic Synthesis Bd. 1-10 (J. Wiley and Sons, Inc.) aufgeführt.

Verbindungen der Formel I, in welcher $R_4$ Wasserstoff, $R_5$ $-COOR_6$ und $R_3$ Fluor bedeuten, können auch aus den entsprechenden Verbindungen der Formel I, in welcher $R_3$ Chlor bedeutet, hergestellt werden; z.B. durch Umsatz mit Alkalifluoriden in geeigneten Lösungsmitteln, gegebenenfalls unter Zusatz von Kronenverbindungen oder Phasentransferkatalysatoren, oder aber durch Umsatz mit wasserfreier Flussäure, Antimonfluorid oder andern geeigneten Reagentien [Angew. Chemie 89, 797 (1977)].

Aus der britischen Patentanmeldung 2 093 448 sind 1-Alkyl-2-acyl-2-phenyl-hydrazincarbonsäure-alkylester bekannt. Diese als Fungizide beschriebenen Verbindungen unterscheiden sich von den erfindungsgemässen insektizid wirksamen 2-Halogencarbonyl-2-phenyl-hydrazincarbonsäure-alkylestern der Formel I hinsichtlich der Substituenten an den N-Atomen und am Phenylring, der bei der in der britischen Patentanmeldung 2 093 448 genannten Verbindungen lediglich mit Methyl-, Äthyl-, Halogen- und Methoxy-Resten substituiert ist. Weiterhin wird in der französischen Patentschrift 2 466 194 zur Bekämpfung von Reis-Zikaden die Verwendung von 2-Alkoxy-4-phenyl-1,3,4-oxadiazolin-5-onen vorgeschlagen, wobei dort jedoch nur das 2-Methoxy-4-(2-methoxyphenyl)-1,3,4-oxadiazolin-5-on spezifisch erwähnt wird. Demgegenüber unterscheiden sich die erfindungsgemässen Oxadiazolinone der Formel I strukturell aufgrund der andersartigen Substitution an der 4-Phenyl-Gruppe.

Es wurde nun überraschenderweise gefunden, dass sich die Verbindungen der Formel I zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina, insbesondere Milben und Zecken, eignen.

Vor allem eignen sich Verbindungen der Formel I zur Bekämpfung von pflanzenschädigenden Insekten in Zier- und Nutzpflanzen, insbesondere in Baumwoll- und Reiskulturen (z.B. Spodoptera littoralis, Heliothis virescens, Nephothettix cincticeps, Chilo, suppressalis und Laodelphax striatellus) sowie Gemüse- und Obstkulturen (z.B. Leptinotarsa decemlineata, Myzus persicae, Laspeyresia pomonella und Adoxophyes reticulana) und von Bodeninsekten (z.B. Aulacophora femoralis, Chortophila brassicae, Diabrotica balteata, Pachnoda savignyi und Scotia ypsilon). Besonders hervorzuheben ist die hohe systemische Wirksamkeit, die vor allem bei der Bekämpfung saugender Insekten zum Tragen kommt.

Wirkstoffe der Formel I zeigen auch eine sehr günstige Wirkung gegen Fliegen, wie z.B. Musca domestica, und Mückenlarven. Ausserdem zeichnen sich die Verbindungen der Formel I durch eine breite, ovizide und ovolarvizide Wirkung aus und besitzen eine wertvolle Wirkung gegen ektoparasitäre Milben und Zecken, z.B. der Familien Ixodidae, Argasidae und Dermanyssidae.

Die Verbindungen der Formel I können als Beizmittel zur Behandlung von Saatgut und Vorrat (Früchte, Knollen, Körner) und Pflanzenstecklingen oder zur Saatfurchenapplikation zum Schutz vor Insekten und Vertretern der Ordnung Akarina, sowie gegen im Erdboden auftretende Insekten eingesetzt werden.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioat, 1,2-Methylendioxy-4-[2-(octylsulfinyl)-propyl]-benzol.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl-oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Essigester, Propylmyristat oder Propylpalmitat, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; Silikonöle oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Öl- oder Stearinsäure, oder von natürlichen Fettsäuregemischen, die z.B. aus Kokosnuss- oder Tallöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäure von Fettalkohol-Äthylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze oder Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Äthylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxid-Addukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien No-nylphenolpolyäthoxyäthanole, Ricinusölpolyglycoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxylalkylreste aufweisen. Die Salze liegen vorzugsweise als Halogenide, Methylsulfate oder Äthylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

«McCutcheon's Detergents and Emulsifiers Annual» MC Publishing Corp., Ridgewood, New Jersey, 1979; Dr. Helmut Stache «Tensid Taschenbuch», Carl Hanser Verlag München/Wien 1981.

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

*Herstellungsbeispiel 1*

Herstellung der Verbindung Nr. 1.1 der Formel

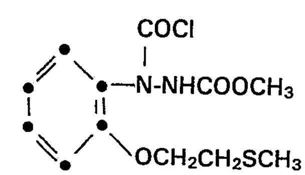

a) Herstellung von 2-(2-Methylthioäthoxy)-nitrobenzol

Zu 8,5 g einer 55%igen NaH Suspension in 150 ml abs. Tetrahydrofuran werden bei 20-25°C 16,3 g 2-Hydroxyäthylmethylsulfid zugetropft. Das Gemisch wird eine Stunde bei 20°C und 20 Minuten bei 40°C gerührt und anschliessend bei 20°C mit 25 g 1-Fluor-2-nitrobenzol versetzt. Nach 10stündigem Rühren der Suspension bei 20°C wird das Tetrahydrofuran im Vakuum abdestilliert. Der Rückstand wird mit Wasser versetzt und mehrmals mit Äther extrahiert. Man erhält ein Öl, welches ohne weitere Reinigung für die nachfolgende Hydrierung eingesetzt werden kann.

b) Herstellung von 2-(2-Methylthioäthoxy)-anilin

33,9 g des bei a) erhaltenen Öls werden in 100 ml abs. Tetrahydrofuran unter Zugabe von 13,6 g Raney-Nickel bei Normaldruck hydriert (Wasserstoffaufnahme = 10,6 Liter). Nach dem Abfiltrieren des Katalysators wird das Filtrat eingedampft und mittels Chromatographie (Kieselgel; Hexan/Essigsäureäthylester als Eluiermittel) gereinigt.

Man erhält 27,4 g Öl, welches für die nachfolgende Reaktion direkt eingesetzt werden kann.

c) Herstellung von 2-(2-Methylthioäthoxy)-phenylhydrazin

27,4 g des unter b) erhaltenen Anilins werden in 45 ml Eisessig vorgelegt und unter Eiskühlung mit einer Mischung von 37 ml konz. HCl und 75 ml $H_2O$ versetzt. Die entstandene Suspension wird bei 0-5°C mit 10,3 g $NaNO_2$ in 22 ml Wasser diazotiert. Die Diazoniumlösung wird 10 Minuten bei 0°C nachgerührt und dann rasch mit einer Lösung von 80,7 g $SnCl_2 \cdot 2H_2O$ in 112 ml konz. HCl unter Kühlung versetzt. Nach vier Stunden wird das Reaktionsgemisch mit Wasser versetzt und mehrmals mit Äther extrahiert. Die wässrige Phase wird anschliessend mit 10%iger wässriger Natronlauge alkalisch gestellt und dreimal mit Äther extrahiert. Nach der üblichen Aufarbeitung der Ätherphasen erhält man die Verbindung der Formel

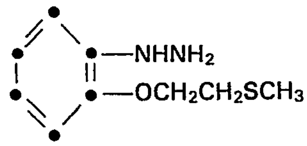

mit einem Schmelzpunkt von 72,5-74,5°C.

d) Herstellung von 2-[2-(Methylthioäthoxy)-phenyl]-hydrazincarbonsäuremethylester

Zu 14,4 g 2-(2-Methylthioäthoxy)-phenylhydrazin in 100 ml Tetrahydrofuran und 14 g N-Äthyldiisopropylamin werden bei 0°C 5,6 ml Chlorameisensäuremethylester zugetropft. Das Reaktionsgemisch wird nach zweistündigem Rühren bei 20°C eingedampft, mit $H_2O$ versetzt und mehrmals mit Essigsäureäthylester extrahiert. Nach der üblichen Aufarbeitung resultiert ein Feststoff, welcher aus Alkohol umkristallisiert wird. Man erhält die Verbindung der Formel

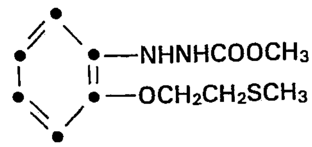

mit einem Schmelzpunkt von 92-94°C.

e) Herstellung von 2-(Chlorcarbonyl)-2-[2-(2-Methylthioäthoxy)-phenyl]-hydrazin-carbonsäuremethylester

14,1 g 2-[2-(2-Methylthioäthoxy)-phenyl]-hydrazincarbonsäuremethylester in 130 ml Toluol werden bei 20°C mit 80 ml einer 20%igen Lösung von Phosgen in Toluol versetzt. Anschliessend wird 3 Stunden auf 90°C erhitzt und das Lösungsmittel entfernt. Nach dem Umkristallisieren des Rückstandes in Äthylalkohol erhält man die Verbindung Nr. 1.1 der Formel

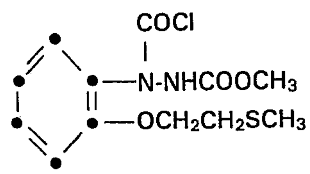

mit einem Schmelzpunkt von 55-57°C.

Auf analoge Weise werden auch folgende Verbindungen hergestellt:

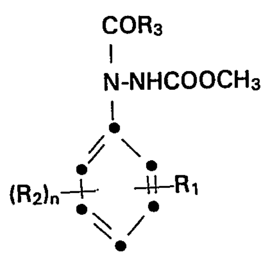

| Nr. | $R_3$ | $R_1$ | $R_2$ | n | Physikalische Daten |
|---|---|---|---|---|---|
| 1.2 | Cl | 2-$OCH_2CH_2Cl$ | H | 1 | NMR ($CDCl_3$): (60 Mhz)<br>$\delta$ = 3,35 s (3H) ($OCH_3$)<br>$\delta$ = 3,7 s (3H) ($COOCH_3$)<br>überlagert von<br>$\delta$ = 3,4-3,8 (m 2H, -$CH_2$-$CH_2$-) |

| Nr. | $R_3$ | $R_1$ | $R_2$ | n | Physikalische Daten |
|---|---|---|---|---|---|
| 1.2 | Cl | 2-$OCH_2CH_2Cl$ | H | 1 | δ = 3,9-4,3 m (2H) (-$CH_2$-$CH_2$-)<br>δ = 6,7-7,65 m (4H)<br>(Phenylprotonen)<br>δ = 8,7 (1H, breit) NH |
| 1.3 | Cl | 2-$OCH_2CH_2OCH_3$ | H | 1 | NMR ($CDCl_3$): (60 Mhz)<br>δ = 3,7 s (H) (-$COOCH_3$)<br>tw. überlagert von<br>δ = 3,6-4,0 m, (2H) (-$CH_2CH_2$-)<br>δ = 4,25 t (2H) (-$CH_2CH_2$-)<br>δ = 6,8-8,0 m (5H)<br>(Phenylprotonen + NH) |
| 1.4 | Cl | O<br>‖<br>2-$OCH_2CH_2SCH_3$<br>‖<br>O | H | 1 | Smp.: 128-130°C |
| 1.5 | Cl | 2-$OCH_2CH_2SCH_2CH_2$ | H | 1 | Smp.: 75-77°C |
| 1.6 | Cl | 2-$OCHCH_2OCH_3$<br>\|<br>$CH_3$ | H | 1 | MS: m/e = 316/318 |
| 1.7 | Cl | 2-$OCH_2CH_2F$ | H | 1 | Smp.: 74-75°C |
| 1.8 | Cl | 4-$OCH_2CH_2SCH_3$ | H | 1 | Smp.: 94-96°C |
| 1.9 | Cl | 2-$OCH_2CH_2CH_2SCH_3$ | H | 1 | MS: m/e = 332/334 |
| 1.10 | Cl | 2-OCH-$CHSCH_3$<br>\| \|<br>$CH_3CH_3$ | H | 1 | MS: m/e = 346/348 |
| 1.11 | Cl | 2-$OCH_2CH_2CH_2OCH_2CH_3$ | H | 1 | MS: m/e = 330/332 |
| 1.12 | Cl | 2-$OCH_2CH_2OCH_2CH_3$ | H | 1 | MS: m/e = 316/318 |
| 1.13 | F | 2-$OCHCH_2OCH_3$<br>\|<br>$CH_3$ | H | 1 | MS: m/e = 300 |
| 1.14 | F | 2-$OCH_2CHOCH_3$<br>\|<br>$CH_3$ | H | ; | MS: m/e = 300 |
| 1.15 | Cl | 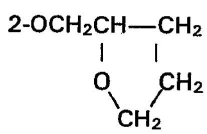 | H | 1 | MS: m/e = 328 |
| 1.16 | Cl | O<br>‖<br>2-$OCH_2CH_2SCH_2CH_3$<br>‖<br>O | H | 1 | Smp.: 101-104°C |
| 1.17 | Cl | 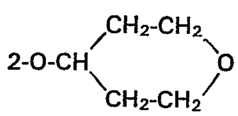 | H | 1 | Smp.: 139-140°C |
| 1.18 | Cl | 2-$OCHCH_2SCH_3$<br>\|<br>$CH_3$ | H | 1 | Smp.: 139-140°C |
| 1.19 | F | 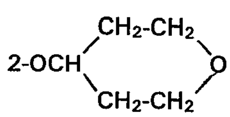 | H | 1 | Smp.: 102-104°C |

| Nr. | $R_3$ | $R_1$ | $R_2$ | n | Physikalische Daten |
|---|---|---|---|---|---|
| 1.20 | Cl | $2\text{-O-CH—(CH}_2)_2$<br>$\text{CH}_2\text{—O}$ | H | 1 | Smp.: 105-107,5°C |
| 1.21 | Cl | $2\text{-O(CH}_2)_2\text{CHOCH}_3$<br>$\text{CH}_3$ | H | 1 | MS: m/e = 330/332 |
| 1.22 | Cl | $2\text{-O(CH}_2)_2\text{OCH}_3$ | $5\text{-CH}_3$ | 1 | Smp.: 48-52°C |
| 1.23 | Cl | $2\text{-OCHCH}_2\text{OCH}_3$<br>$\text{CH}_3$ | $5\text{-CH}_3$ | 1 | Smp.: 73-75°C |
| 1.24 | F | $2\text{-OCHCH}_2\text{OCH}_3$<br>$\text{CH}_3$ | $5\text{-CH}_3$ | 1 | Smp.: 63-67°C |
| 1.25 | Cl | $2\text{-O(CH}_2)_2\text{SCH}_3$ | $5\text{-CH}_3$ | 1 | Smp.: 110-111°C |
| 1.26 | F | $2\text{-O(CH}_2)_2\text{OCH}_3$ | $5\text{-CH}_3$ | 1 | Smp.: 71-73°C |
| 1.27 | Cl | $2\text{-OCH-CH}_2\text{OCH}_3$<br>$\text{CH}_3$ | $6\text{-CH}_3$ | 1 | Smp.: 83-85°C |
| 1.28 | Cl | $2\text{-OCHCH}_2\text{OCH}_3$<br>$\text{CH}_3$ | $3,5\text{-CH}_3$ | 2 | Smp.: 83-86°C |
| 1.29 | Cl | $2\text{-OCH}_2\text{CHOCH}_3$<br>$\text{CH}_3$ | H | 1 | MS: m/e = 316/318 |
| 1.30 | F | $2\text{-OCHCH}_2\text{OCH}_3$<br>$\text{CH}_3$ | $3,5\text{-CH}_3$ | 2 | Smp.: 83-85°C |
| 1.31 | Cl | $2\text{-OCHCH}_2\text{OCH}_3$<br>$\text{CH}_2\text{OCH}_3$ | H | 1 | MS: m/e = 346/348 |
| 1.32 | F | $2\text{-OCHCH}_2\text{OCH}_3$<br>$\text{CH}_2\text{OCH}_3$ | H | 1 | $n_D^{23°}$ = 1,4962 |
| 1.33 | F | $2\text{-O-CH}_2\text{CH—CH}_2$<br>$\text{CH}_2\ \ \text{CH}_2$<br>O | H | 1 | Smp.: 90-92°C |

Herstellung der Verbindung Nr. 2.1 der Formel

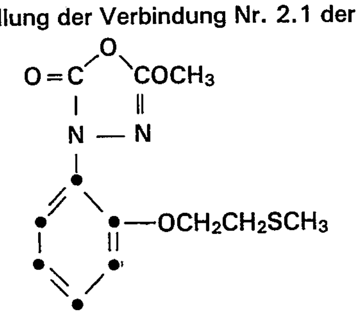

5,8 g 2-(Chlorcarbonyl)-2-[2-(methylthioäthoxy)-phenyl]-hydrazincarbonsäuremethylester (Verbindung Nr. 1.1) in 45 ml Methanol suspendiert werden bei 20°C mit 5 ml Triäthylamin versetzt. Nach 5stündigem Rühren bei 20°C wird das Reaktionsgemisch mit Wasser verdünnt und mit Äther extrahiert. Die organische Phase wird abgetrennt, mit Wasser und mit verdünnter Kochsalzlösung gewaschen, filtriert, über Magnesiumsulfat getrocknet und eingeengt. Nach dem Chromatographieren des Produktes an Kieselgel mit Toluol/Essigester (95 : 5) als Eluiermittel, erhält man die Titelverbindung Nr. 2.1 mit einem Schmelzpunkt von 52-53°C.

Auf analoge Weise werden auch folgende Verbindungen hergestellt:

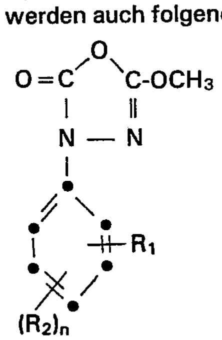

| Nr. | $R_1$ | $R_2$ | n | Physikalische Daten |
|---|---|---|---|---|
| 2.2 | 2-$OCH_2CH_2OCH_3$ | H | 1 | $n_D^{21°}$ = 1,5260 |
| 2.3 | 2-$OCH_2CH_2Cl$ | H | 1 | Smp.: 75-78,5°C |
| 2.4 | 2-$SCH_2CH_2OCH_3$ | H | 1 | $n_D^{25°}$ = 1,560 |
| 2.5 | 2-$OCH_2CH_2SCH_3$ | H | 1 | Smp.: 73-75°C |
| 2.6 | 1-$OCH_2CH_2SCH_2CH_3$ | H | 1 | Smp.: 41-43°C |
| 2.7 | 2-$OCHCH_2OCH_3$<br>\|<br>$CH_3$ | H | 1 | $n_D^{22°}$ = 1,5177 |
| 2.8 | 2-$OCH_2CH_2F$ | H | 1 | Smp.: 55-57°C |
| 2.9 | 2-$OCH_2CH_2SOCH_3$ | H | 1 | Smp.: 119-121°C |
| 2.10 | 2-$OCH_2CH_2SO_2CH_3$ | H | 1 | Smp.: 105-107°C |
| 2.11 | 2-$OCH_2CH_2OCH_2CH_3$ | H | 1 | Smp. 44-46°C |
| 2.12 | 2-$OCH_2CH_2CH_2OCH_2CH_3$ | H | 1 | $n_D^{21°}$ = 1,5152 |
| 2.13 | 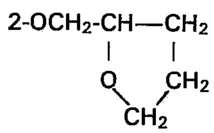 | H | 1 | MS: m/e = 292 |
| 2.14 | 2-$OCH_2CH_2SO_2CH_2CH_3$ | H | 1 | Smp.: 89-91°C |
| 2.15 | 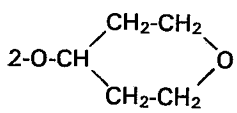 | H | 1 | MS: m/e = 292 |
| 2.16 | 2-$OCHCH_2SO_2CH_3$<br>\|<br>$CH_3$ | H | 1 | Smp.: 134-136°C |
| 2.17 | 2-$OCHCH_2SCH_3$<br>\|<br>$CH_3$ | H | 1 | $n_D^{24°}$ = 1,5459 |
| 2.18 | 2-$OCH$-$CHSCH_3$<br>\| \|<br>$CH_3CH_3$ | H | 1 | MS: m/e = 280 |
| 2.19 | 2-$O(CH_2)_2CHOCH_3$<br>\|<br>$CH_3$ | H | 1 | $n_D^{23°}$ = 1,5156 |
| 2.20 | 2-$O(CH_2)_2OCH_3$ | 5-$CH_3$ | 1 | $n_D^{23°}$ = 1,5232 |
| 2.21 | 2-$OCHCH_2OCH_3$<br>\|<br>$CH_3$ | 5-$CH_3$ | 1 | $n_D^{23°}$ = 1,5171 |
| 2.22 | 2-$O(CH_2)_2CHOCH_3$<br>\|<br>$CH_3$ | H | 1 | $n_D^{23°}$ = 1,5156 |
| 2.23 | 2-$O(CH_2)_2SCH_3$ | 5-$CH_3$ | 1 | $n_D^{23°}$ = 1,5520 |
| 2.24 | 2-$OCH_2CHOCH_3$<br>\|<br>$CH_3$ | H | 1 | $n_D^{23°}$ = 1,5189 |
| 2.25 | 2-$OCHCH_2OCH_3$<br>\|<br>$CH_3$ | 3,5-$CH_3$ | 2 | $n_D^{24°}$ = 1,5160 |
| 2.26 | 2-$O(CH_2)_2SO_2CH_3$ | 5-$CH_3$ | 1 | Smp.: 134-136°C |

| Nr. | $R_1$ | $R_2$ | n | Physikalische Daten |
|---|---|---|---|---|
| 2.27 | $2\text{-}OCHCH_2OCH_3$<br>I<br>$CH_3$ | $6\text{-}CH_3$ | 1 | $n_D^{23°} = 1{,}5131$ |
| 2.28 | $2\text{-}OCHCH_2OCH_3$<br>I<br>$CH_2OCH_3$ | H | 1 | Smp.: 72-73°C |
| 2.29 | $2\text{-}OCH_2CF_3$ | H | 1 | Smp.: 57-59°C |

*Beispiel 2*

*Formulierungsbeispiele für Wirkstoffe gemäss dem Herstellungsbeispiel 1*

(% = Gewichtsprozent)

| 2.1 *Emulsions-Konzentrate* | a) | b) | c) | d) |
|---|---|---|---|---|
| Wirkstoff gemäss dem Herstellungsbeispiel 1 | 10% | 25% | 40% | 50% |
| Ca-Dodecylbenzolsulfat | — | 5% | 8% | 6% |
| Ricinusöl-polyäthylenglykoläther (36 Mol AeO) | — | 5% | — | — |
| Tributylphenol-polyäthylenglykoläther (30 Mol AeO) | — | — | 12% | 4% |
| Ricinusölthioxilat | 25% | — | — | — |
| Cyclohexanon | — | — | 15% | 20% |
| Butanol | 15% | — | — | — |
| Xylolgemisch | — | 65% | 25% | 20% |
| Essigester | 50% | — | — | — |

Aus solchen Konzentraten können durch Verdünnen mit Wasser Emulsionen jeder gewünschten Konzentration hergestellt werden.

| 2.2 *Lösungen* | a) | b) |
|---|---|---|
| Wirkstoff gemäss dem Herstellungsbeispiel 1 | 10% | 5% |
| Äthylenglykol-monomethyläther | — | — |
| Polyäthylenglykol (MG 400) | 70% | — |
| N-Methyl-2-pyrrolidon | 20% | — |
| Epoxidiertes Kokosnussöl | — | 1% |
| Benzin (Siedegrenzen 160-190°C) | — | 94% |

Die Lösungen sind zur Anwendung in Form kleinster Tropfen geeignet.

| 2.3 *Granulate* | a) | b) |
|---|---|---|
| Wirkstoff gemäss dem Herstellungsbeispiel 1 | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend im Vakuum abgedampft.

| 2.4 *Stäubemittel* | a) | b) | c) | d) |
|---|---|---|---|---|
| Wirkstoff gemäss dem Herstellungsbeispiel 1 | 2% | 5% | 5% | 8% |
| Hochdisperse Kieselsäure | 1% | 5% | — | — |
| Talkum | 97% | — | 95% | — |
| Kaolin | — | 90% | — | 92% |

Durch inniges Vermischen der Trägerstoffe mit dem Wirkstoff erhält man gebrauchsfertige Stäubemittel.

| 2.5 *Spritzpulver* | a) | b) | c) |
|---|---|---|---|
| Wirkstoff gemäss dem Herstellungsbeispiel 1 | 20% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyäthylenglykoläther (7-8 Mol AeO) | — | 2% | — |
| Hochdisperse Kieselsäure | 5% | 10% | 10% |
| Kaolin | 67% | 27% | — |

Der Wirkstoff wird mit den Zusatzstoffen gut vermischt und in einer geeigneten Mühle gut vermahlen. Man erhält Spritzpulver, die sich mit Wasser zur Suspensionen jeder gewünschten Konzentration verdünnen lassen.

| 2.6 *Extruder Granulat* | |
|---|---|
| Wirkstoff gemäss dem Herstellungsbeispiel 1 | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.7 *Umhüllungs-Granulat* | |
|---|---|
| Wirkstoff gemäss dem Herstellungsbeispiel 1 | 3% |
| Polyäthylenglykol (MG 200) | 3% |
| Kaolin | 94% |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

2.8 *Suspensions-Konzentrat*

| | |
|---|---|
| Wirkstoff gemäss dem Herstellungsbeispiel 1 | 40% |
| Äthylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässerige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

*Beispiel 3*

*Biologiebeispiele für Wirkstoffe gemäss dem Herstellungsbeispiel 1*

3.1 *Insektizide Frassgift-Wirkung: Spodoptera littoralis*

Baumwollpflanzen werden mit einer Versuchslösung, enthaltend 400 ppm der zu prüfenden Verbindung, besprüht.

Nach dem Antrocknen des Belages werden die Pflanzen mit Larven von Spodoptera littoralis ($L_1$-Stadium) besetzt. Man verwendet pro Versuchsverbindung zwei Pflanzen. Die Auswertung der erzielten Abtötungsrate erfolgt nach 2, 4, 24, 48 und 72 Stunden. Der Versuch wird bei 28°C und 60% relativer Luftfeuchtigkeit durchgeführt.

Verbindungen gemäss Beispiel 1 zeigen im obigen Test 80-100%ige Wirkung gegenüber Spodoptera littoralis-Larven.

3.2 *Wirkung gegen Lucilia sericata*

Zu 9 ml eines Zuchtmediums wird bei 50°C ein ml einer 0,1% Aktivsubstanz enthaltenden wässrigen Zubereitung hinzugefügt. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss Beispiel 1 zeigen in diesem Test eine 80-100%ige Wirkung gegen Lucilia sericata.

3.3 *Wirkung gegen Aëdes aegypti*

Auf die Oberfläche von 150 ml Wasser, das sich in einem Behälter befindet, wird so viel einer 0,1%igen acetonischen Lösung des Wirkstoffs pipettiert, dass eine Konzentration von 12,5 ppm erhalten wird. Nach Verdunsten des Acetons wird der Behälter mit 30 bis 40 2tägigen Aëdes-Larven beschickt. Nach 2 und 7 Tagen wird die % Mortalität (Anzahl der schwimmunfähigen Larven) geprüft.

Verbindungen gemäss Beispiel 1 zeigen 100%ige Wirkung (Mortalität) im obigen Test.

3.4 *Ovizide Wirkung auf Heliothis virescens*

Entsprechende Mengenanteile einer benetzbaren pulverförmigen Formulierung, enthaltend 25 Gew.-% des zu prüfenden Wirkstoffes, werden mit jeweils so viel Wasser vermischt, dass sich wässrige Emulsionen mit Wirkstoffkonzentrationen von 400 bis 12,5 ppm ergeben.

In diese wirkstoffhaltigen Emulsionen werden eintägige Eigelege von Heliothis auf Cellophan® während drei Minuten eingetaucht und dann auf Raumfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgelegt. Zur Auswertung wird die zur 100%igen Abtötung der Eier erforderliche Wirkstoffkonzentration bestimmt.

Verbindungen gemäss Beispiel 1 zeigt bei 12,5 ppm 100%ige Wirkung (Mortalität) im obigem Test.

3.5 *Wirkung gegen pflanzenschädigende Akariden: Tetranychus urticae (OP-sensible) und Tetranychus cinnabarinus (OP-tolerant)*

Die Primärblätter von Phaseolus vulgaris-Pflanzen werden 16 Stunden vor dem Versuch auf akarizide Wirkung mit einem infestierten Blattstück aus einer Massenzucht von Tetranychus urticae (OP-sens.) bzw. Tetranychus cinnabarinus (OP-tol.) belegt. (Die Toleranz bezieht sich auf die Verträglichkeit gegenüber Diazinon.)

Die so behandelten infestierten Pflanzen werden mit einer Versuchslösung enthaltend 400 ppm der zu prüfenden Verbindung bis zur Tropfnässe besprüht.

Nach 24 Stunden und wiederum nach 7 Tagen werden Imagines und Larven (alle beweglichen Stadien) unter dem Binokular auf lebende und tote Individuen ausgewertet. Man verwendet pro Konzentration und pro Testspezies eine Pflanze. Während des Versuchsverlaufs stehen die Pflanzen in Gewächshauskabinen bei 25°C.

Verbindungen gemäss Beispiel 1 zeigen in diesem Versuch eine 80-100%ige Wirkung gegen Tetranychus urticae und Tetranychus cinnabarinus.

3.6 *Insektizide Kontaktwirkung: Aphis craccivora*

In Töpfen angezogene Bohnen-Pflanzen (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 50, 12,5 oder 3 ppm der zu prüfenden Verbindung bis zur Tropfnässe besprüht. Man verwendet pro Test-Verbindung und pro Konzentration zwei Pflanzen, und eine Auswertung der erzielten Abtötungsrate erfolgt nach weiteren 24 Stunden.

Verbindungen gemäss Beispiel 1 zeigen im obigen Test die in der folgenden Tabelle angegebene Wirkung gegen Aphis craccivora.

*Biologische Versuchsergebnisse*

In der folgenden Tabelle sind Versuchsergebnisse auf der Basis des vorstehenden Beispiels ausgeführt, und zwar mit folgendem Bewertungsindex in bezug auf die prozentuale Abtötung der Schädlinge:

A: >80% Abtötung bei 50 ppm

B: >80% Abtötung bei 12,5 ppm

C: >80% Abtötung bei 3 ppm

| Verbindung Nr. | Wirkung gegen Aphis craccivora |
|---|---|
| 1.1 | D |
| 1.2 | A |
| 1.3 | B |
| 2.1 | B |
| 2.2 | A |
| 2.3 | C |
| 2.12 | B |
| 2.13 | A |

3.7 *Insektizide Kontaktwirkung: Myzus persicae*

Etwa 4 cm hohe, in Wasser angezogene Erbsenkeimlinge werden vor Versuchsbeginn je mit ca. 200 Individuen der Spezies Myzus persicae besiedelt. Die so behandelten Pflanzen werden 24 Stunden später mit einer wässrigen Suspension enthaltend 400 ppm der zu prüfenden Verbindung bis zur Tropfnässe besprüht. Man verwendet pro Konzentration zwei Pflanzen. Eine Auswertung der erzielten Abtötungsrate erfolgt 48 Stunden nach Applikation. Der Versuch wird bei 20-22°C und 60% relativer Luftfeuchtigkeit durchgeführt.

Verbindungen der Formel I gemäss Beispiel 1 zeigen in diesem Test eine 80-100%ige Wirkung.

3.8 *Wirkung gegen Bodeninsekten (Diabrotica balteata)*

Es werden 5 etwa einen bis drei cm lange Maiskeimlinge sowie eine Rondelle aus Filterpapier in eine 0,2 bis 12,5 ppm des zu prüfenden Wirkstoffes enthaltende wässrige Zubereitung getaucht. Die feuchte Filterpapierrondelle wird auf dem Boden eines 200 ml Plastikbechers ausgelegt, und dann werden die 5 behandelten Maiskeimlinge zusammen mit 10 Larven von Diabrotica balteata des zweiten bis dritten Larvalstadiums in den Becher gegeben. Pro Wirkstoffkonzentration werden 2 Ansätze durchgeführt. Die mit den Larven besetzten Becher werden während 6 Tagen bei Tageslicht, einer relativen Luftfeuchtigkeit von 40 bis 60% und einer Temperatur von 22 bis 24°C gehalten. Danach wird die prozentuale Abtötung der Testtiere bestimmt.

Verbindungen gemäss Beispiel 1 zeigen in diesem Test 80-100%ige Wirkung.

3.9 *Wirkung gegen Zecken*

A Amblyomma hebraeum

50 Nymphen werden in ein Glasröhrchen gezählt und für 1 bis 2 Minuten in 2 ml einer wässrigen Emulsion aus einer Verdünnungsreihe mit je 10, 1 oder 0,1 ppm Testsubstanz getaucht. Das Röhrchen wird dann mit einem genormten Wattebausch verschlossen und auf den Kopf gestellt, damit die Wirkstoffemulsion von der Watte aufgenommen werden kann.

Die Auswertung erfolgt nach 1 Woche. Für jeden Versuch werden 2 Wiederholungen durchgeführt.

B) Boophilus microplus (Larven)

Mit einer analogen Verdünnungsreihe wie beim Test A) werden mit je 20 sensiblen resp. OP-resistenten Larven Versuche durchgeführt. (Die Resistenz bezieht sich auf die Verträglichkeit von Diazinon).

Die Verbindungen gemäss dem Herstellungsbeispiel 1 zeigen eine 80-100%ige Wirkung gegen Nymphen bzw. Larven der Zecken: Amblyomma hebraeum und Boophilus microplus.

3.10 *Insektizide Wirkung: Nilaparvata lugens*

Reispflanzen werden mit einer Versuchslösung, enthaltend 400 ppm der zu prüfenden Verbindung, besprüht. Nach dem Antrocknen des Belages werden die Pflanzen mit Nymphen von Nilaparvata lugens (N2 oder N3-Stadium) besetzt. Man verwendet pro Versuchsverbindung und pro Test-Spezies zwei Pflanzen. Die Auswertung der erzielten Abtötungsrate erfolgt nach 6 Tagen. Der Versuch wird bei 26°C und 60% relativer Luftfeuchtigkeit durchgeführt.

Verbindungen gemäss Beispiel 1 zeigen im obigen Test 100% Wirkung gegenüber Nilaparvata lugens-Nymphen.

3.11 *Systemische insektizide Wirkung: Nilaparvata lugens*

Reispflanzen werden mit 5 ml einer Versuchslösung, enthaltend 50, 12,5 oder 3 ppm der zu prüfenden Verbindung, angegossen und nach 1, 2 resp. 3 Wochen mit jeweils 20 Nymphen besiedelt. Die Auswertung der erzielten Abtötungsrate erfolgt 6 Tage nach der Besiedlung. Verbindungen gemäss Beispiel 1 zeigen 80-100%ige Wirkung in diesem Test.

**Patentansprüche**

1. Ein Phenylhydrazin der Formel I

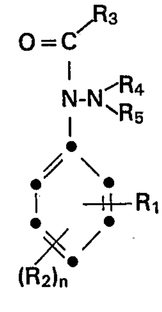

(I)

worin

$R_1$ -A-R-Z,

A Sauerstoff, Schwefel, $-\overset{O}{\overset{\|}{S}}-$ oder $-\overset{O}{\overset{\|}{\underset{\underset{O}{\|}}{S}}}-$,

R $C_1$-$C_6$-Alkylen, $C_1$-$C_6$-Halogenalkyl-$C_1$-$C_6$-alkylen, $C_1$-$C_6$-Alkoxy-$C_1$-$C_6$-alkylen oder $C_1$-$C_6$-Alkylthio-$C_1$-$C_6$-alkylen,

Z Halogen, $C_1$-$C_6$-Halogenalkyl, $C_1$-$C_6$-Alkoxy, $C_1$-$C_6$-Alkylthio, $C_1$-$C_6$-Alkylsulfinyl oder $C_1$-$C_6$-Alkylsulfonyl, oder

R und Z zusammen 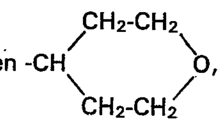

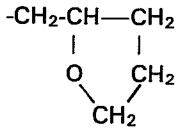 oder 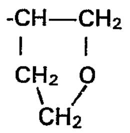 ,

$R_2$ Wasserstoff, Halogen, $C_1$-$C_6$-Alkyl, $C_1$-$C_6$-Alkoxy, $C_1$-$C_6$-Alkylthio oder $(C_1$-$C_6$-Alkyl$)_2$amino,

n die Zahlen 1-4, wobei $R_2$ verschieden sein kann, falls n eine der Zahlen 2-4 ist,

$R_3$ Halogen,

$R_4$ Wasserstoff,

$R_5$ $-COOR_6$ oder

$R_3$, $R_4$ und $R_5$ zusammen ein Brückenglied der Formel $-O-\underset{\underset{O}{\|}}{C}-OR_6$

und

$R_6$ $C_1$-$C_6$-Alkyl, $C_2$-$C_6$-Alkenyl oder $C_2$-$C_6$-Alkinyl bedeuten.

2. Eine Verbindung gemäss Anspruch 1 der Formel Ia

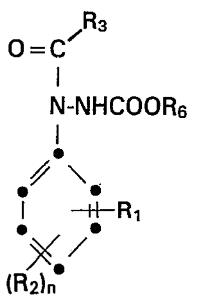

(Ia)

worin

$R_1$ -A-R-Z,

A Sauerstoff, Schwefel, $-\overset{O}{\overset{\|}{S}}-$ oder $-\overset{O}{\overset{\|}{\underset{\underset{O}{\|}}{S}}}-$,

R $C_1$-$C_6$-Alkylen, $C_1$-$C_6$-Halogenalkyl-$C_1$-$C_6$-alkylen, $C_1$-$C_6$-Alkoxy-$C_1$-$C_6$-alkylen oder $C_1$-$C_6$-Alkylthio-$C_1$-$C_6$-alkylen,

Z Halogen, $C_1$-$C_6$-Halogenalkyl, $C_1$-$C_6$-Alkoxy, $C_1$-$C_6$-Alkylthio, $C_1$-$C_6$-Alkylsulfinyl oder $C_1$-$C_6$-Alkylsulfonyl, oder

R und Z zusammen 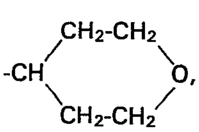

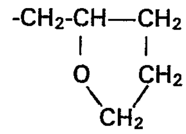 oder 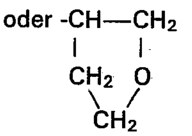 ,

$R_2$ Wasserstoff, Halogen, $C_1$-$C_6$-Alkyl, $C_1$-$C_6$-Alkoxy, $C_1$-$C_6$-Alkylthio oder $(C_1$-$C_6$-Alkyl$)_2$amino,

n die Zahlen 1-4, wobei $R_2$ verschieden sein kann, falls n eine der Zahlen 2-4 ist,

$R_3$ Halogen,

und

$R_6$ $C_1$-$C_6$-Alkyl, $C_2$-$C_6$-Alkenyl oder $C_2$-$C_6$-Alkinyl bedeuten.

3. Eine Verbindung gemäss Anspruch 1 der Formel Ib

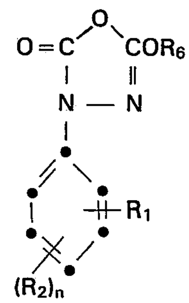

(Ib)

worin

$R_1$ -A-R-Z,

A Sauerstoff, Schwefel, $-\overset{O}{\overset{\|}{S}}-$ oder $-\overset{O}{\overset{\|}{\underset{\underset{O}{\|}}{S}}}-$,

R $C_1$-$C_6$-Alkylen, $C_1$-$C_6$-Halogenalkyl-$C_1$-$C_6$-alkylen, $C_1$-$C_6$-Alkoxy-$C_1$-$C_6$-alkylen oder $C_1$-$C_6$-Alkylthio-$C_2$-$C_6$-alkylen,

Z Halogen, $C_1$-$C_6$-Halogenalkyl, $C_1$-$C_6$-Alkoxy, $C_1$-$C_6$-Alkylthio, $C_1$-$C_6$-Alkylsulfinyl oder $C_1$-$C_6$-Alkylsulfonyl,

R und Z zusammen

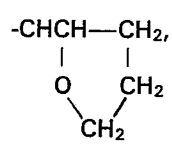, 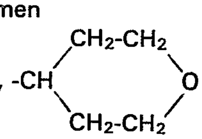 oder

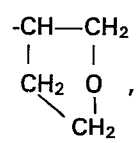 ,

$R_2$ Wasserstoff, Halogen, $C_1$-$C_6$-Alkyl, $C_1$-$C_6$-Alkoxy, $C_1$-$C_6$-Alkylthio oder $(C_1$-$C_6$-Alkyl$)_2$amino,

n die Zahlen 1-4, wobei $R_2$ verschieden sein kann, falls n eine der Zahlen 2-4 ist, und

$R_6$ $C_1$-$C_6$-Alkyl, $C_2$-$C_6$-Alkenyl oder $C_2$-$C_6$-Alkinyl bedeuten.

4. Eine Verbindung gemäss Anspruch 2, worin

$R_1$ -A-R-Z,

A Sauerstoff, Schwefel, $-\underset{\underset{O}{\|}}{S}-$ oder $-\overset{O}{\overset{\|}{\underset{\underset{O}{\|}}{S}}}-$,

R $C_2$-$C_6$-Alkylen,
Z Halogen, $C_1$-$C_6$-Halogenalkyl, $C_1$-$C_6$-Alkoxy, $C_1$-$C_6$-Alkylthio, $C_1$-$C_6$-Alkylsulfinyl oder $C_1$-$C_6$-Alkylsulfonyl oder

R und Z zusammen

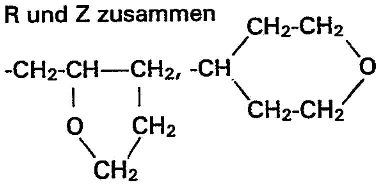 oder

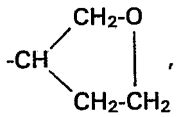 ,

$R_2$ Wasserstoff, Halogen, $C_1$-$C_6$-Alkyl, $C_1$-$C_6$-Alkoxy, $C_1$-$C_6$-Alkylthio oder $(C_1$-$C_6$-Alkyl$)_2$amino,
n die Zahlen 1-4, wobei $R_2$ verschieden sein kann, falls n eine der Zahlen 2-4 ist,
$R_3$ Halogen und
$R_6$ $C_1$-$C_6$-Alkyl, $C_2$-$C_6$-Alkenyl oder $C_2$-$C_6$-Alkinyl bedeuten.

5. Eine Verbindung gemäss Anspruch 4, worin
$R_1$ -A-R-Z,
A Sauerstoff oder Schwefel,
$R_1$ unsubstituiertes $C_2$-$C_6$-Alkylen,
Z Halogen, $C_1$-$C_6$-Alkoxy, $C_1$-$C_6$-Alkylthio, $C_1$-$C_6$-Alkylsulfinyl oder $C_1$-$C_6$-Alkylsulfonyl,
$R_2$ Wasserstoff, Halogen oder $C_1$-$C_6$-Alkyl,
n die Zahl 1,
$R_3$ Halogen und
$R_6$ Methyl bedeuten.

6. Eine Verbindung gemäss Anspruch 5, worin
$R_1$ -A-R-Z in 2-Position zur

Gruppe 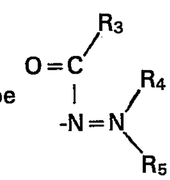 steht,

A Sauerstoff,
R unsubstituiertes $C_2$-$C_4$-Alkylen,
Z Halogen, $C_1$-$C_4$-Alkoxy, $C_1$-$C_4$-Alkylthio, $C_1$-$C_4$-Alkylsulfinyl oder $C_1$-$C_4$-Alkylsulfonyl,
$R_2$ Wasserstoff, Halogen oder $C_1$-$C_4$-Alkyl,
n die Zahl 1,
$R_3$ Halogen und
$R_6$ Methyl bedeuten.

7. Eine Verbindung gemäss den Ansprüchen 2, 4, 5 und 6, worin $R_3$ Fluor bedeutet.

8. Eine Verbindung gemäss Anspruch 3, worin
$R_1$ -A-R-Z,
A Sauerstoff, Schwefel, 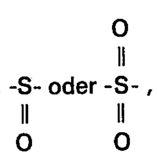 ,
R $C_2$-$C_6$-Alkylen,
Z Halogen, $C_1$-$C_6$-Halogenalkyl, $C_1$-$C_6$-Alkoxy, $C_1$-$C_6$-Alkylthio, $C_1$-$C_6$-Alkylsulfinyl oder $C_1$-$C_6$-Alkylsulfonyl oder

R und Z zusammen

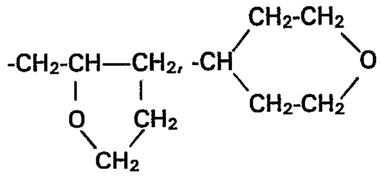 oder

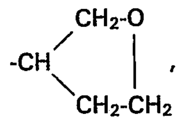 ,

$R_2$ Wasserstoff, Halogen, $C_1$-$C_6$-Alkyl, $C_1$-$C_6$-Alkoxy, $C_1$-$C_6$-Alkylthio oder $(C_1$-$C_6$-Alkyl$)_2$amino,
n die Zahlen 1-4, wobei $R_2$ verschieden sein kann, falls n eine der Zahlen 2-4 ist, und
$R_6$ $C_1$-$C_6$-Alkyl, $C_2$-$C_6$-Alkenyl oder $C_2$-$C_6$-Alkinyl bedeuten.

9. Eine Verbindung gemäss Anspruch 8, worin

A Sauerstoff, Schwefel, 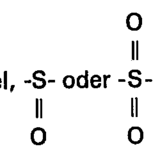 ,
R $C_2$-$C_6$-Alkylen,
Z Halogen, $C_1$-$C_6$-Alkoxy, $C_1$-$C_6$-Alkylthio, $C_1$-$C_6$-Alkylsulfinyl oder $C_1$-$C_6$-Alkylsulfonyl,
$R_2$ Wasserstoff, Halogen, $C_1$-$C_6$-Alkoxy, $C_1$-$C_6$-Alkylthio oder $(C_1$-$C_6$-Alkyl$)_2$amino,
n die Zahlen 1-4, wobei $R_2$ verschieden sein kann, falls n eine der Zahlen 2-4 ist, und
$R_6$ $C_1$-$C_6$-Alkyl, $C_2$-$C_6$-Alkenyl oder $C_2$-$C_6$-Alkinyl bedeuten.

10. Eine Verbindung gemäss Anspruch 9, worin
$R_1$ -A-R-Z,
A Sauerstoff oder Schwefel,
R $C_2$-$C_6$-Alkylen,
Z Halogen, $C_1$-$C_6$-Alkoxy, $C_1$-$C_6$-Alkylthio, $C_1$-$C_6$-Alkylsulfinyl oder $C_1$-$C_6$-Alkylsulfonyl,
n die Zahl 1 und
$R_6$ Methyl bedeuten.

11. Eine Verbindung gemäss Anspruch 10, worin
$R_1$ -A-R-Z in 2-Position zur

Gruppe 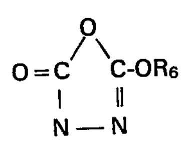 steht,

A Sauerstoff,
R unsubstituiertes $C_2$-$C_4$-Alkylen,
Z Halogen, $C_1$-$C_4$-Alkoxy, $C_1$-$C_4$-Alkylthio, $C_1$-$C_4$-Alkylsulfinyl oder $C_1$-$C_4$-Alkylsulfonyl,
$R_2$ Wasserstoff, Halogen oder $C_1$-$C_4$-Alkyl,
n die Zahl 1
und
$R_6$ Methyl bedeuten.

12. Die Verbindung gemäss Anspruch 6 der Formel

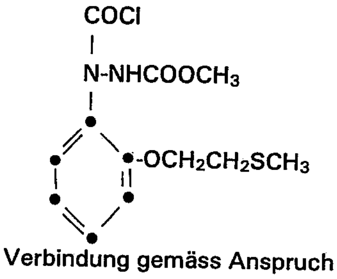

13. Die Verbindung gemäss Anspruch 6 der Formel

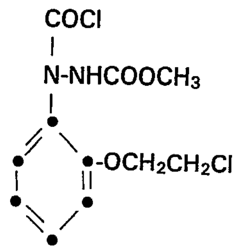

14. Die Verbindung gemäss Anspruch 6 der Formel

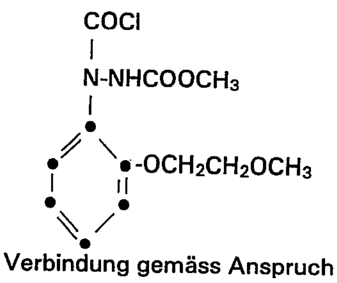

15. Die Verbindung gemäss Anspruch 5 der Formel

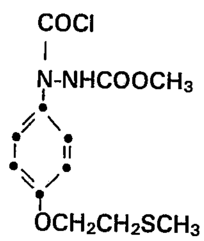

16. Die Verbindung gemäss Anspruch 6 der Formel

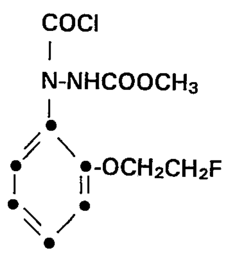

17. Die Verbindung gemäss Anspruch 6 der Formel

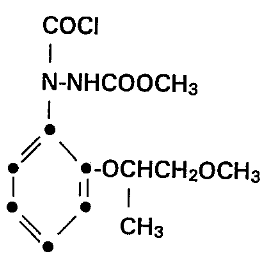

18. Die Verbindung gemäss Anspruch 6 der Formel

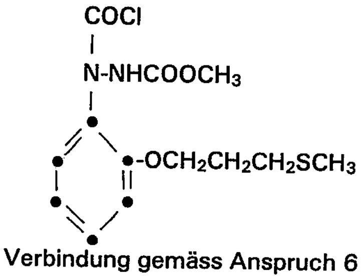

19. Die Verbindung gemäss Anspruch 6 der Formel

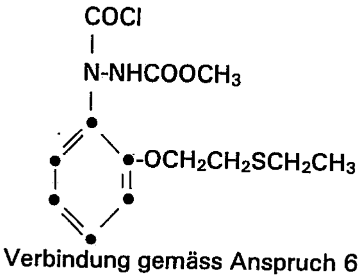

20. Die Verbindung gemäss Anspruch 6 der Formel

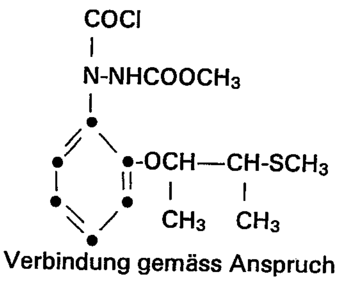

21. Die Verbindung gemäss Anspruch 6 der Formel

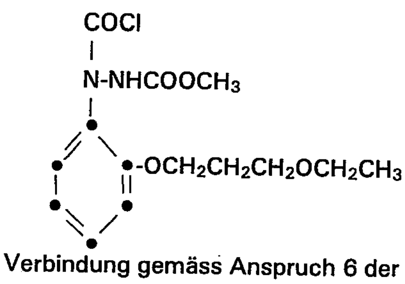

22. Die Verbindung gemäss Anspruch 6 der Formel

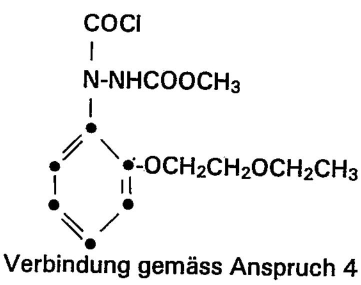

23. Die Verbindung gemäss Anspruch 4 der Formel

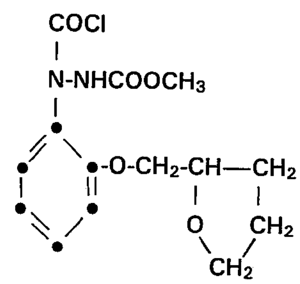

24. Die Verbindung gemäss Anspruch 4 der Formel

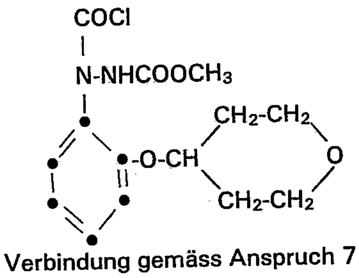

25. Die Verbindung gemäss Anspruch 7 der Formel

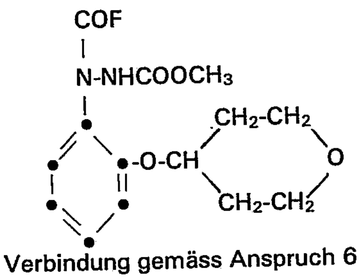

26. Die Verbindung gemäss Anspruch 6 der Formel

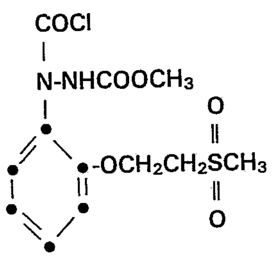

27. Die Verbindung gemäss Anspruch 6 der Formel

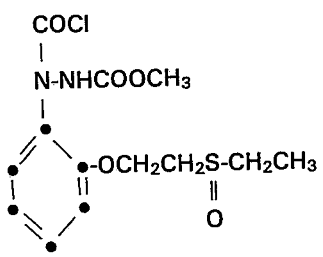

28. Die Verbindung gemäss Anspruch 6 der Formel

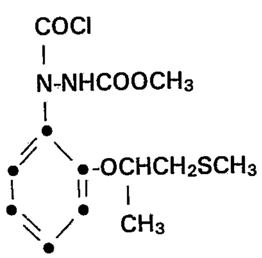

29. Die Verbindung gemäss Anspruch 7 der Formel

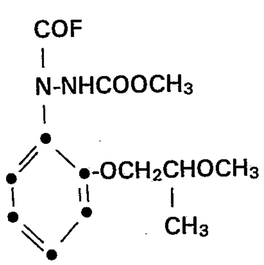

30. Die Verbindung gemäss Anspruch 7 der Formel

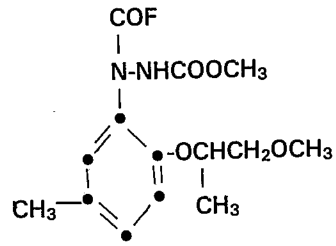

31. Die Verbindung gemäss Anspruch 7 der Formel

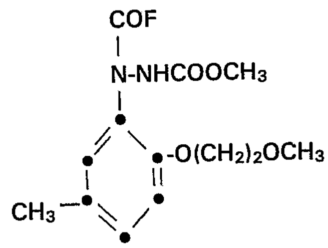

32. Die Verbindung gemäss Anspruch 7 der Formel

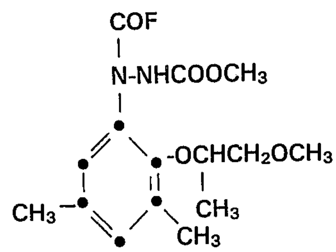

33. Die Verbindung gemäss Anspruch 7 der Formel

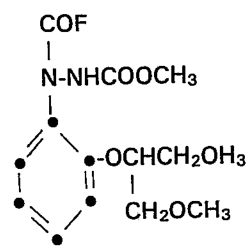

34. Die Verbindung gemäss Anspruch 7 der Formel

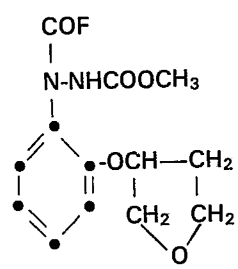

35. Die Verbindung gemäss Anspruch 11 der Formel

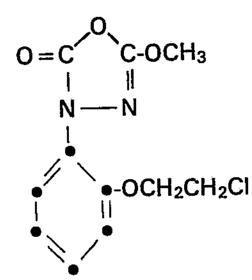

36. Die Verbindung gemäss Anspruch 11 der Formel

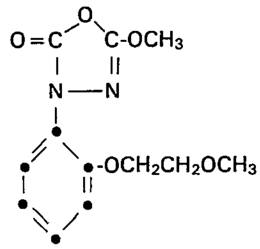

37. Die Verbindung gemäss Anspruch 11 der Formel

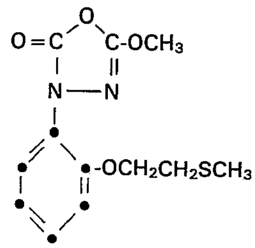

38. Die Verbindung gemäss Anspruch 11 der Formel

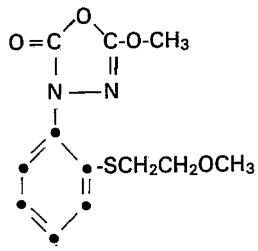

39. Die Verbindung gemäss Anspruch 11 der Formel

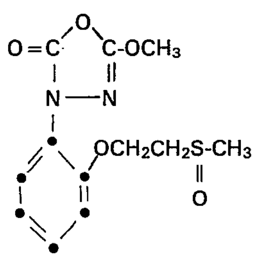

40. Die Verbindung gemäss Anspruch 11 der Formel

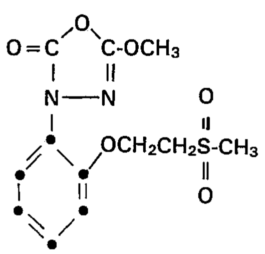

41. Die Verbindung gemäss Anspruch 11 der Formel

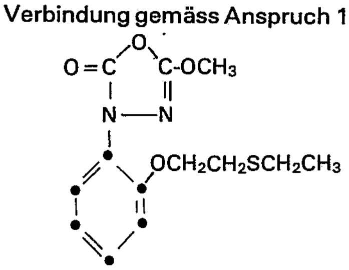

42. Die Verbindung gemäss Anspruch 11 der Formel

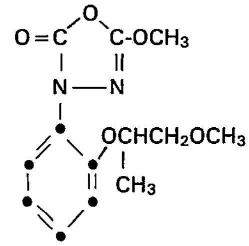

43. Die Verbindung gemäss Anspruch 11 der Formel

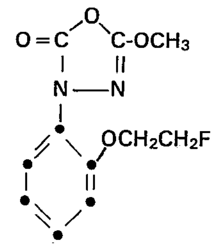

44. Die Verbindung gemäss Anspruch 11 der Formel

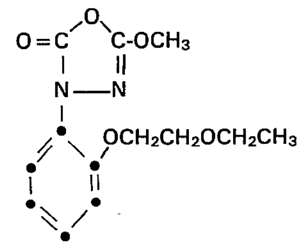

45. Die Verbindung gemäss Anspruch 8 der Formel

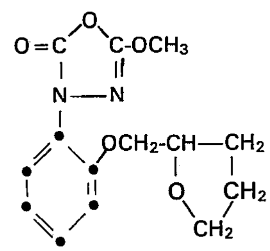

46. Die Verbindung gemäss Anspruch 11 der Formel

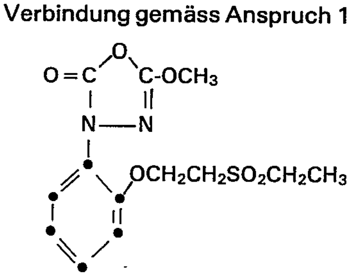

47. Die Verbindung gemäss Anspruch 8 der Formel

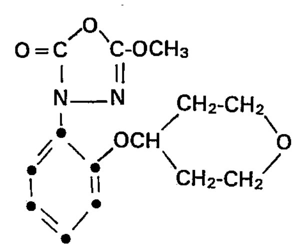

48. Die Verbindung gemäss Anspruch 11 der Formel

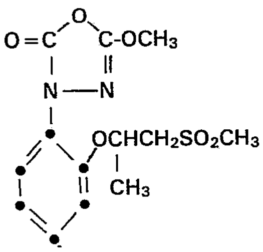

49. Die Verbindung gemäss Anspruch 11 der Formel

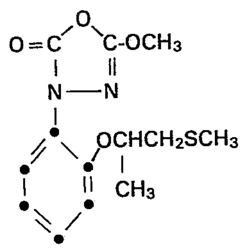

50. Die Verbindung gemäss Anspruch 3 der Formel

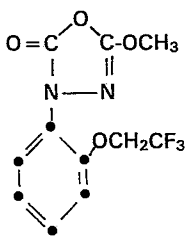

51. Die Verbindung gemäss Anspruch 11 der Formel

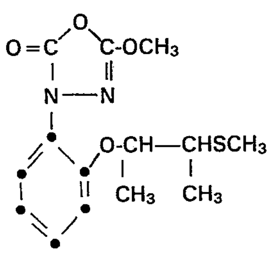

52. Die Verbindung gemäss Anspruch 10 der Formel

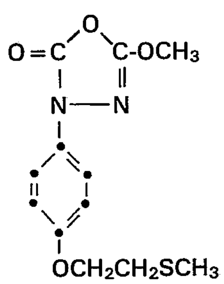

53. Die Verbindung gemäss Anspruch 11 der Formel

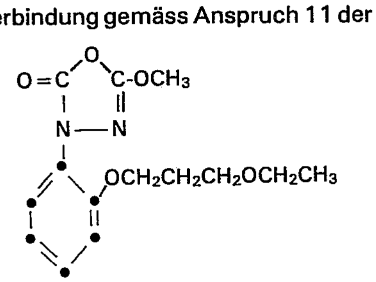

54. Die Verbindung gemäss Anspruch 3 der Formel

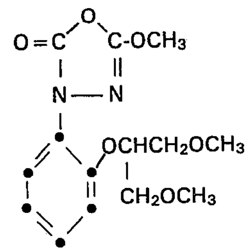

55. Verfahren zur Herstellung von Verbindungen der Formel I gemäss Anspruch 1, dadurch gekennzeichnet, dass man eine Verbindung der Formel II

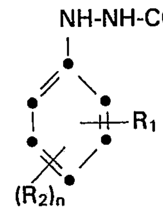 (II)

worin $R_1$, $R_2$, $R_6$ und n die für die Formel I angegebene Bedeutung haben, in einem ersten Verfahrensschritt mit einer Verbindung der Formel III

$CO(R_3)_2$ (III)

worin $R_3$ Halogen bedeutet umsetzt und anschliessend an den entstandenen Verbindungen der Formel IV

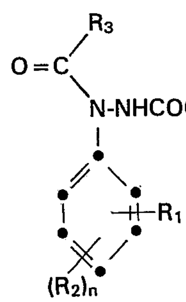 (IV)

worin $R_1$, $R_2$, $R_3$, $R_6$ und n die für die Formel I angegebene Bedeutung haben in Gegenwart einer Base die Ringschlussreaktion durchführt.

56. Ein Schädlingsbekämpfungsmittel, dadurch gekennzeichnet, dass es neben geeigneten Träger- und/oder anderen Zuschlagstoffen als aktive Komponente eine Verbindung gemäss Anspruch 1 enthält.

57. Die Verwendung von Verbindungen gemäss Anspruch 1 zur Bekämpfung von verschiedenenartigen Schädlingen an Tieren und Pflanzen sowie im Boden.

58. Die Verwendung gemäss Anspruch 57 zur Bekämpfung von Insekten und Vertretern der Ordnung Akarina.

59. Verfahren zur Bekämpfung von Schädlingen an Tieren und Pflanzen sowie im Boden, dadurch gekennzeichnet, dass man eine Verbindung gemäss Anspruch 1 auf die Tiere, die Pflanzen oder den Boden appliziert.

**Claims**

1. A phenylhydrazine of the formula I

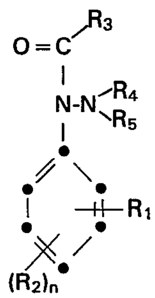

(I)

wherein

$R_1$ is -A-R-Z,

A is oxygen, sulfur, 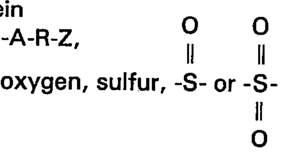

R $C_1$-$C_6$-alkylene, $C_1$-$C_6$-haloalkyl-$C_1$-$C_6$-alkylene, $C_1$-$C_6$-alkoxy-$C_1$-$C_6$-alkylene or $C_1$-$C_6$-alkylthio-$C_1$-$C_6$-alkylene,

Z is halogen, $C_1$-$C_6$-haloalkyl, $C_1$-$C_6$-alkoxy, $C_1$-$C_6$-alkylthio, $C_1$-$C_6$-alkylsulfinyl or $C_1$-$C_6$-alkylsulfonyl, or

R and Z together are 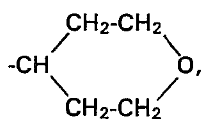

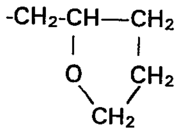 oder 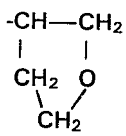 ,

$R_2$ is hydrogen, halogen, $C_1$-$C_6$-alkyl, $C_1$-$C_6$-alkoxy, $C_1$-$C_6$-alkylthio or ($C_1$-$C_6$-alkyl)$_2$amino,

n is a number from 1-4, and $R_2$ can be different when n is one of the numbers 2-4,

$R_3$ is halogen,

$R_4$ is hydrogen,

$R_5$ is -$COOR_6$, or

$R_3$, $R_4$ and $R_5$ together form a bridge member of the formula 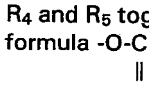, and

$R_6$ is $C_1$-$C_6$-alkyl, $C_2$-$C_6$-alkenyl or $C_2$-$C_6$-alkynyl.

2. A compound according to claim 1 of the formula Ia

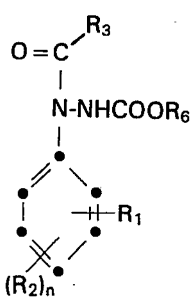

(Ia)

wherein

$R_1$ is -A-R-Z,

A is oxygen, sulfur, 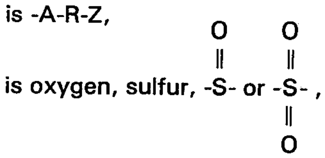

R is $C_1$-$C_6$-alkylene, $C_1$-$C_6$-haloalkyl-$C_1$-$C_6$-alkylene, $C_1$-$C_6$-alkoxy-$C_1$-$C_6$-alkylene or $C_1$-$C_6$-alkylthio-$C_1$-$C_6$-alkylene,

Z is halogen, $C_1$-$C_6$-haloalkyl, $C_1$-$C_6$-alkoxy, $C_1$-$C_6$-alkylthio, $C_1$-$C_6$-alkylsulfinyl or $C_1$-$C_6$-alkylsulfonyl, or

R and Z together are 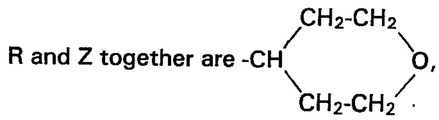

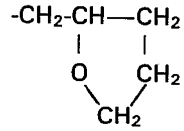 oder 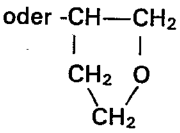 ,

$R_2$ is hydrogen, halogen, $C_1$-$C_6$-alkyl, $C_1$-$C_6$-alkoxy, $C_1$-$C_6$-alkylthio or ($C_1$-$C_6$-alkyl)$_2$amino,

n is a number from 1-4, and $R_2$ can be different when n is one of the numbers 2-4,

$R_3$ is halogen, and

$R_6$ is $C_1$-$C_6$-alkyl, $C_2$-$C_6$-alkenyl or $C_2$-$C_6$-alkynyl.

3. A compound according to claim 1 of the formula Ib

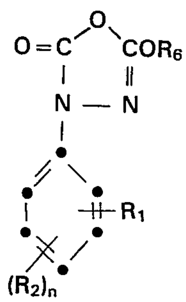

(Ib)

wherein

$R_1$ is -A-R-Z,

A is oxygen, sulfur, 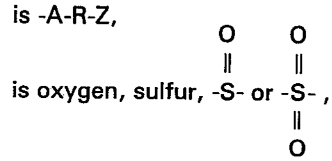

R is $C_1$-$C_6$-alkylene, $C_1$-$C_6$-haloalkyl-$C_1$-$C_6$-alkylene, $C_1$-$C_6$-alkoxy-$C_1$-$C_6$-alkylene or $C_1$-$C_6$-alkylthio-$C_2$-$C_6$-alkylene,

Z is halogen, $C_1$-$C_6$-haloalkyl, $C_1$-$C_6$-alkoxy, $C_1$-$C_6$-alkylthio, $C_1$-$C_6$-alkylsulfinyl or $C_1$-$C_6$-alkylsulfonyl, or

R and Z together are

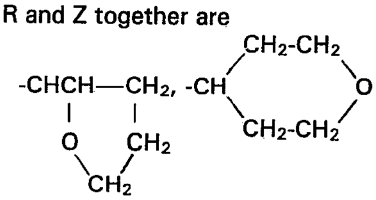 or

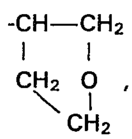 ,

$R_2$ is hydrogen, halogen $C_1$-$C_6$-alkyl, $C_1$-$C_6$-alkoxy, $C_1$-$C_6$-alkylthio, or ($C_1$-$C_6$-alkyl)$_2$amino,

n is a number from 1-4, and $R_2$ can be different when n is one of the numbers 2-4, and

$R_6$ is $C_1$-$C_6$-alkyl, $C_2$-$C_6$-alkenyl or $C_2$-$C_6$-alkynyl.

4. A compound according to claim 2 wherein

$R_1$ is -A-R-Z,

A is oxygen, sulfur, 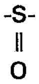 or 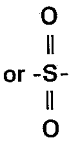 ,

R is $C_2$-$C_6$-alkylene,

Z is halogen, $C_1$-$C_6$-haloalkyl, $C_1$-$C_6$-alkoxy, $C_1$-$C_6$-alkylthio, $C_1$-$C_6$-alkylsulfinyl or $C_1$-$C_6$-alkylsulfonyl or

R and Z together are

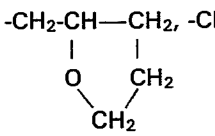 , 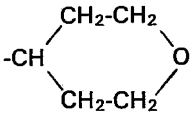 or

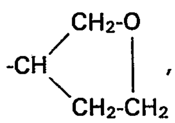 ,

$R_2$ is hydrogen, halogen, $C_1$-$C_6$-alkyl, $C_1$-$C_6$-alkoxy, $C_1$-$C_6$-alkylthio or ($C_1$-$C_6$-alkyl)$_2$amino,

n is a number from 1-4, and $R_2$ can be different when n is one of the numbers 2-4,

$R_3$ is halogen, and

$R_6$ is $C_1$-$C_6$-alkyl, $C_2$-$C_6$-alkenyl or $C_2$-$C_6$-alkynyl.

5. A compound according to claim 4 wherein

$R_1$ is -A-R-Z,

A is oxygen or sulfur,

$R_1$ is unsubstituted $C_2$-$C_6$-alkylene,

Z is halogen, $C_1$-$C_6$-alkoxy, $C_1$-$C_6$-alkylthio, $C_1$-$C_6$-alkylsulfinyl or $C_1$-$C_6$-alkylsulfonyl,

$R_2$ is hydrogen, halogen or $C_1$-$C_6$-alkyl,

n is the number 1,

$R_3$ is halogen, and

$R_6$ is methyl.

6. A compound according to claim 5 wherein

$R_1$ is -A-R-Z in the 2-position with respect to the group

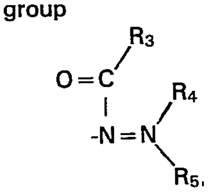 ,

A is oxygen,

R is unsubstituted $C_2$-$C_4$-alkylene,

Z is halogen, $C_1$-$C_4$-alkoxy, $C_1$-$C_4$-alkylthio, $C_1$-$C_4$-alkylsulfinyl or $C_1$-$C_4$-alkylsulfonyl,

$R_2$ is hydrogen, halogen or $C_1$-$C_4$-alkyl,

n is the number 1,

$R_3$ is halogen, and

$R_6$ is methyl.

7. A compound according to claims 2, 4, 5 and 6 wherein $R_3$ is fluorine.

8. A compound according to claim 3 wherein

$R_1$ is -A-R-Z,

A is oxygen, sulfur, 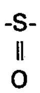 or 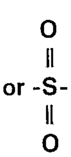 ,

R is $C_2$-$C_6$-alkylene,

Z is halogen, $C_1$-$C_6$-haloalkyl, $C_1$-$C_6$-alkoxy, $C_1$-$C_6$-alkylthio, $C_1$-$C_6$-alkylsulfinyl or $C_1$-$C_6$-alkylsulfonyl, or

R and Z together are

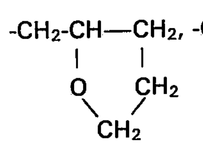 , 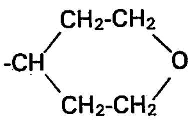 or

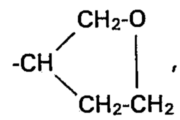 ,

$R_2$ is hydrogen, halogen, $C_1$-$C_6$-alkyl, $C_1$-$C_6$-alkoxy, $C_1$-$C_6$-alkylthio or ($C_1$-$C_6$-alkyl)$_2$amino,

n is a number from 1-4, and $R_2$ can be different when n is one of the numbers 2-4, and

$R_6$ is $C_1$-$C_6$-alkyl, $C_2$-$C_6$-alkenyl or $C_2$-$C_6$-alkynyl.

9. A compound according to claim 8 wherein

A is oxygen, sulfur, 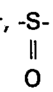 or 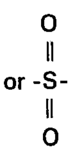 ,

R is $C_2$-$C_6$-alkylene,

Z is halogen, $C_1$-$C_6$-alkoxy, $C_1$-$C_6$-alkylthio, $C_1$-$C_6$-alkylsulfinyl or $C_1$-$C_6$-alkylsulfonyl,

$R_2$ is hydrogen, halogen, $C_1$-$C_6$-alkoxy, $C_1$-$C_6$-alkylthio or ($C_1$-$C_6$-alkyl)$_2$amino,

n is a number from 1-4, and $R_2$ can be different when n is one of the numbers 2-4, and

$R_6$ is $C_1$-$C_6$-alkyl, $C_2$-$C_6$-alkenyl or $C_2$-$C_6$-alkynyl.

10. A compound according to claim 9 wherein

$R_1$ is -A-R-Z,

A is oxygen or sulfur,

R is $C_2$-$C_6$-alkylene,

Z is halogen, $C_1$-$C_6$-alkoxy, $C_1$-$C_6$-alkylthio, $C_1$-$C_6$-alkylsulfinyl or $C_1$-$C_6$-alkylsulfonyl,

n is the number 1 and

$R_6$ is methyl.

11. A compound according to claim 10 wherein

$R_1$ is -A-R-Z in the 2-position with respect to the group

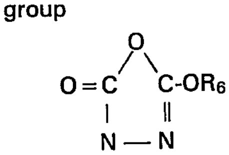 ,

A is oxygen,

R is unsubstituted $C_2$-$C_4$-alkylene,

Z is halogen, $C_1$-$C_4$-alkoxy, $C_1$-$C_4$-alkylthio, $C_1$-$C_4$-alkylsulfinyl or $C_1$-$C_4$-alkylsulfonyl,

$R_2$ is hydrogen, halogen or $C_1$-$C_4$-alkyl,

n is the number 1, and

$R_6$ is methyl.

12. The compound according to claim 6 of the formula

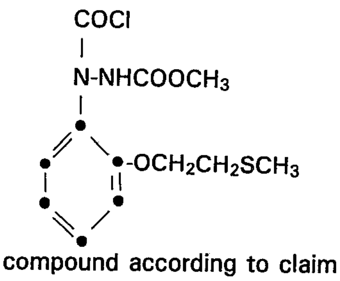

13. The compound according to claim 6 of the formula

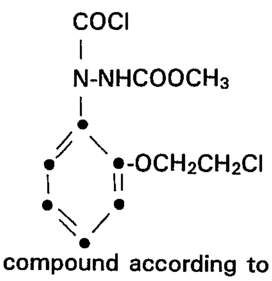

14. The compound according to claim 6 of the formula

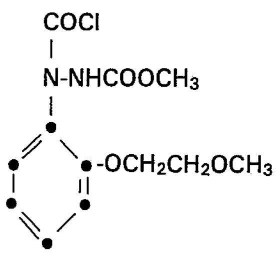

15. The compound according to claim 5 of the formula

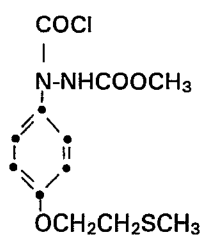

16. The compound according to claim 6 of the formula

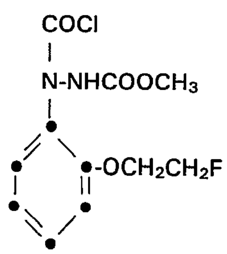

17. The compound according to claim 6 of the formula

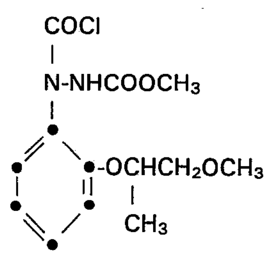

18. The compound according to claim 6 of the formula

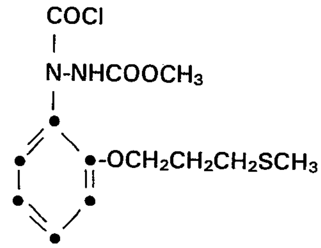

19. The compound according to claim 6 of the formula

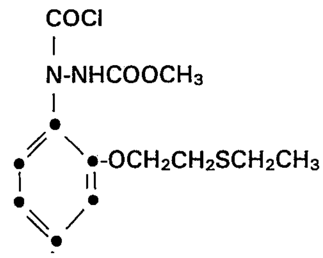

20. The compound according to claim 6 of the formula

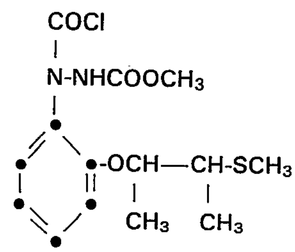

21. The compound according to claim 6 of the formula

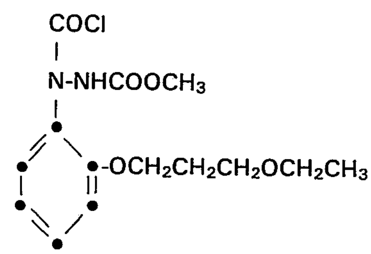

22. The compound according to claim 6 of the formula

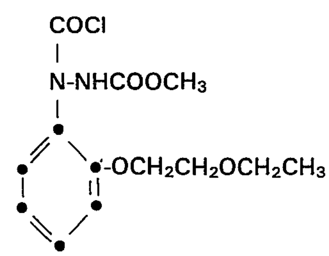

23. The compound according to claim 4 of the formula

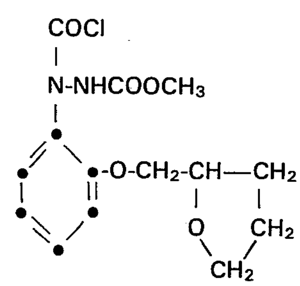

24. The compound according to claim 4 of the formula

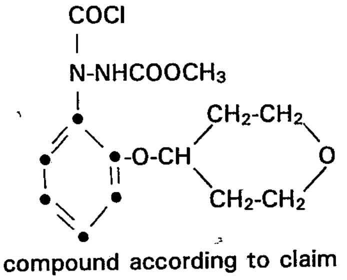

25. The compound according to claim 7 of the formula

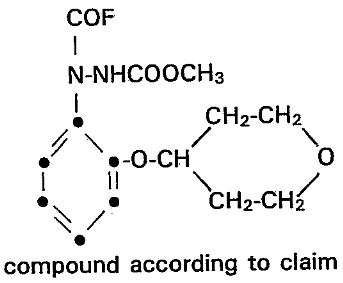

26. The compound according to claim 6 of the formula

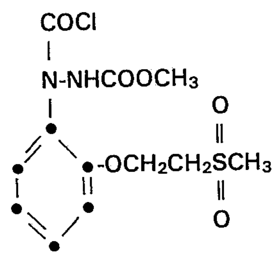

27. The compound according to claim 6 of the formula

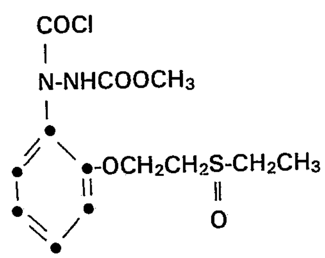

28. The compound according to claim 6 of the formula

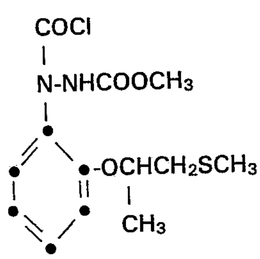

29. The compound according to claim 7 of the formula

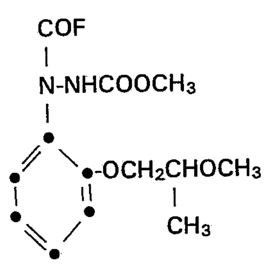

30. The compound according to claim 7 of the formula

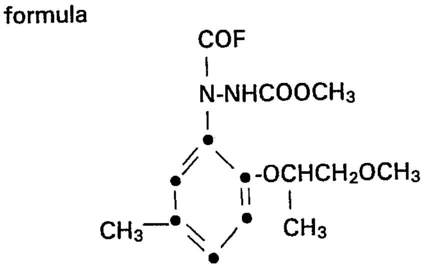

31. The compound according to claim 7 of the formula

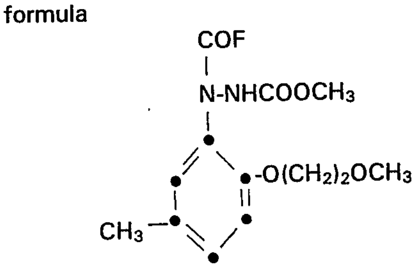

32. The compound according to claim 7 of the formula

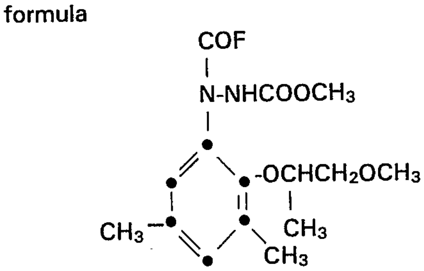

33. The compound according to claim 7 of the formula

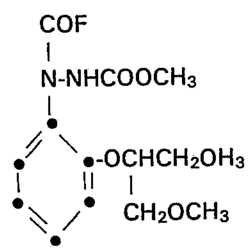

34. The compound according to claim 7 of the formula

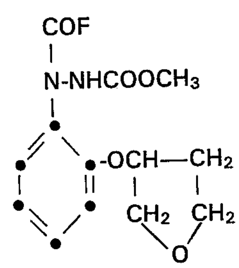

35. The compound according to claim 11 of the formula

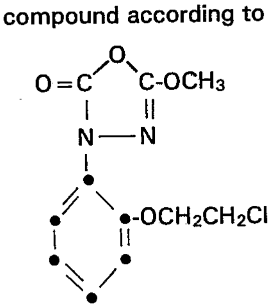

36. The compound according to claim 11 of the formula

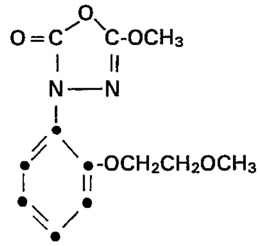

37. The compound according to claim 11 of the formula

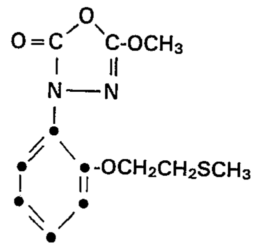

38. The compound according to claim 11 of the formula

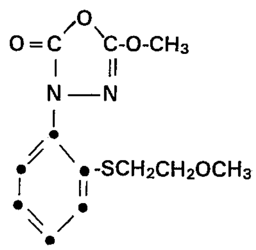

39. The compound according to claim 11 of the formula

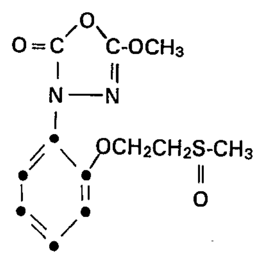

40. The compound according to claim 11 of the formula

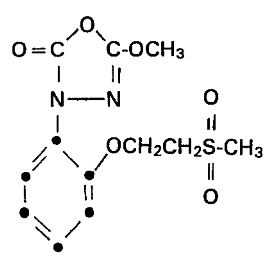

41. The compound according to claim 11 of the formula

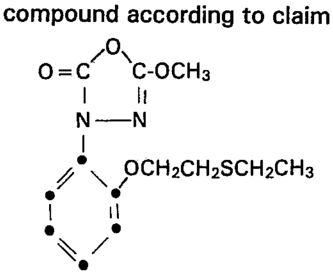

42. The compound according to claim 11 of the formula

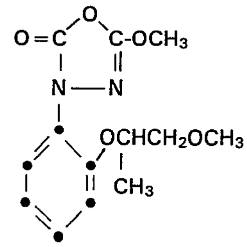

43. The compound according to claim 11 of the formula

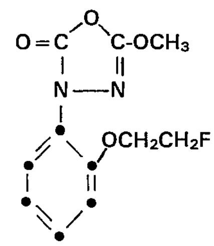

44. The compound according to claim 11 of the formula

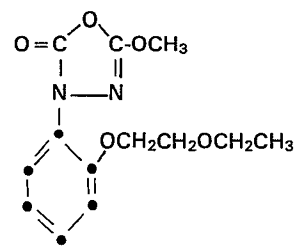

45. The compound according to claim 8 of the formula

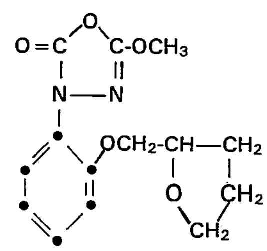

46. The compound according to claim 11 of the formula

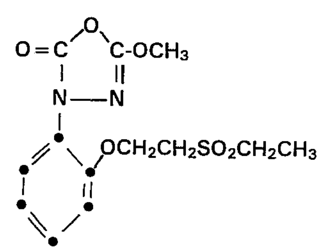

47. The compound according to claim 8 of the formula

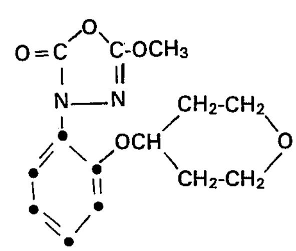

48. The compound according to claim 11 of the formula

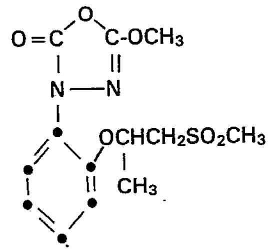

49. The compound according to claim 11 of the formula

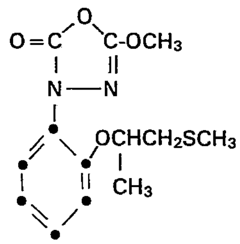

50. The compound according to claim 3 of the formula

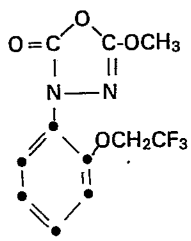

51. The compound according to claim 11 of the formula

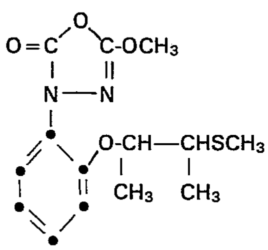

52. The compound according to claim 10 of the formula

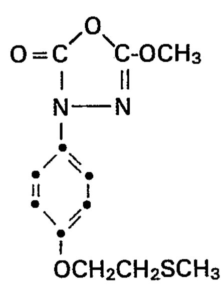

53. The compound according to claim 11 of the formula

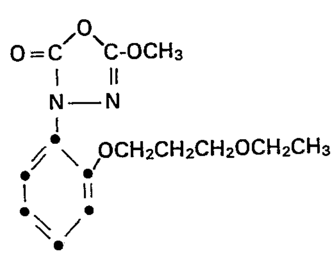

54. The compound according to claim 3 of the formula

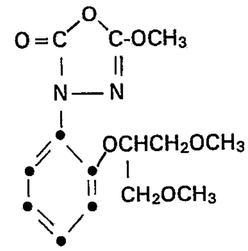

55. Process for producing compounds of the formula I according to claim 1, characterised in that in a first step a compound of the formula II

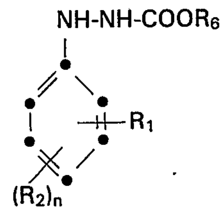

(II)

wherein $R_1$, $R_2$, $R_6$ and n have the meanings defined under the formula I, is reacted with a compound of the formula

$$CO(R_3)_2 \qquad (III)$$

wherein $R_3$ is halogen; and subsequently the ring closure reaction is carried out in the presence of a base on the formed compounds of the formula IV

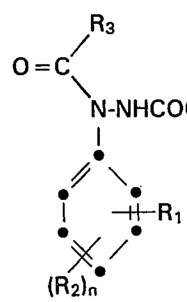

(IV)

wherein $R_1$, $R_2$, $R_3$, $R_6$ and n have the meanings defined under the formula I.

56. A pesticidal composition, characterised in that it contains as active ingredient a compound according to claim 1, together with suitable carriers and/or other additives.

57. The use of compounds according to claim 1 for controlling various pests on animals and plants and in the soil.

58. The use according to claim 57 for controlling insects, and members of the order Acarina.

59. A process for controlling pests on animals and plants and in the soil, characterised in that a compound according to claim 1 is applied to the animals, to the plants or to the soil.

**Revendications**

1. Phénylhydrazine de formule I

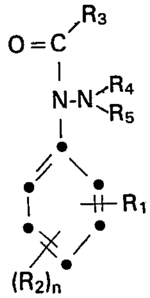

I

où

$R_1$ représente -A-R-Z,

A représente un oxygène, soufre, 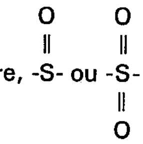 ,

R représente un alcoylène en $C_1$ à $C_6$, halogène-alcoyle en $C_1$ à $C_6$-alcoylène en $C_1$ à $C_6$, alcoxy en $C_1$ à $C_6$-alcoylène en $C_1$ à $C_6$ ou alcoylthio en $C_1$ à $C_6$-alcoylène en $C_1$ à $C_6$,

Z représente un halogène, halogènealcoyle en $C_1$ à $C_6$, alcoxy en $C_1$ à $C_6$, alcoylthio en $C_1$ à $C_6$, alcoylsulfinyle en $C_1$ à $C_6$ ou alcoylsulfonyle en $C_1$ à $C_6$, ou

R et Z représentent ensemble 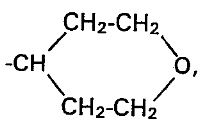,

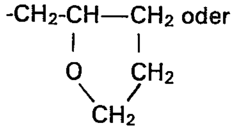 oder 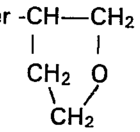 ,

$R_2$ représente un hydrogène, halogène, alcoyle en $C_1$ à $C_6$, alcoxy en $C_1$ à $C_6$, alcoylthio en $C_1$ à $C_6$ ou (alcoyle en $C_1$ à $C_6$)$_2$amino,

n représente les nombres 1 à 4, où $R_2$ peut être différent si n est l'un des nombres 2-4,

$R_3$ représente un halogène,

$R_4$ représente un hydrogène,

$R_5$ représente $-COOR_6$ ou

$R_3$, $R_4$ et $R_5$ représentent ensemble un élément de pont de formule 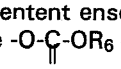 et

$R_6$ représente un alcoyle en $C_1$ à $C_6$, alcényle en $C_2$ à $C_6$ ou alcynyle en $C_2$ à $C_6$.

2. Composé selon la revendication 1 de formule Ia

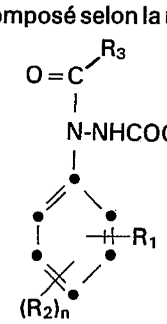

(Ia)

$R_1$ représente -A-R-Z,

A représente un oxygène, soufre, 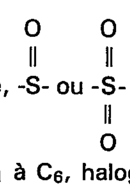 ,

R représente un alcoylène en $C_1$ à $C_6$, halogène-alcoyle en $C_1$ à $C_6$-alcoylène en $C_1$ à $C_6$, alcoxy en $C_1$ à $C_6$-alcoylène en $C_1$ à $C_6$ ou alcoylthio en $C_1$ à $C_6$-alcoylène en $C_1$ à $C_6$,

Z représente un halogène, halogènealcoyle en $C_1$ à $C_6$, alcoxy en $C_1$ à $C_6$, alcoylthio en $C_1$ à $C_6$, alcoylsulfinyle en $C_1$ à $C_6$ ou alcoylsulfonyle en $C_1$ à $C_6$, ou

R et Z représentent ensemble 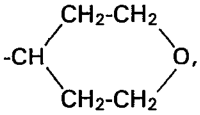,

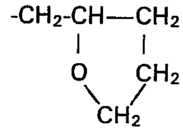 oder 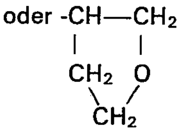 ,

$R_2$ représente un hydrogène, halogène, alcoyle en $C_1$ à $C_6$, alcoxy en $C_1$ à $C_6$, alcoylthio en $C_1$ à $C_6$ ou (alcoyle en $C_1$ à $C_6$)$_2$amino,

n représente les nombres 1 à 4, où $R_2$ peut être différent si n est l'un des nombres 2-4,

$R_3$ représente un halogène, et

$R_6$ représente un alcoyle en $C_1$ à $C_6$, alcényle en $C_2$ à $C_6$ ou alcynyle en $C_2$ à $C_6$.

3. Composé selon la revendication 1 de formule Ib

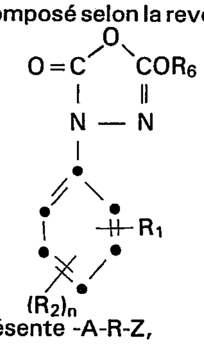

(Ib)

$R_1$ représente -A-R-Z,

A représente un oxygène, soufre, 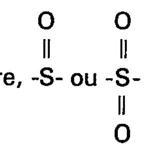 ,

R représente un alcoylène en $C_1$ à $C_6$, halogène-alcoyle en $C_1$ à $C_6$-alcoylène en $C_1$ à $C_6$, alcoxy en $C_1$ à $C_6$-alcoylène en $C_1$ à $C_6$ ou alcoylthio en $C_1$ à $C_6$-alcoylène en $C_2$ à $C_6$,

Z représente un halogène, halogènealcoyle en $C_1$ à $C_6$, alcoxy en $C_1$ à $C_6$, alcoylthio en $C_1$ à $C_6$, alcoylsulfinyle en $C_1$ à $C_6$ ou alcoylsulfonyle en $C_1$ à $C_6$,

R et Z représentent ensemble

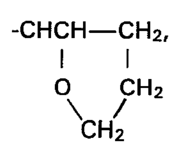, 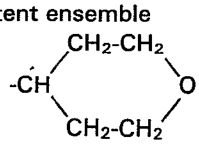 ou

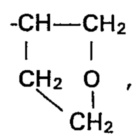 ,

$R_2$ représente un hydrogène, halogène, alcoyle en $C_1$ à $C_6$, alcoxy en $C_1$ à $C_6$, alcoylthio en $C_1$ à $C_6$ ou (alcoyle en $C_1$ à $C_6$)$_2$amino,

n représente les nombres 1 à 4, où $R_2$ peut être différent si n est l'un des nombres 2-4, et

$R_6$ représente un alcoyle en $C_1$ à $C_6$, alcényle en $C_2$ à $C_6$ ou alcynyle en $C_2$ à $C_6$.

4. Composé selon la revendication 2, où
$R_1$ représente -A-R-Z,

A représente un oxygène, soufre, 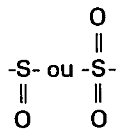

R représente un alcoylène en $C_2$ à $C_6$,
Z représente un halogène, halogènealcoyle en $C_1$ à $C_6$, alcoxy en $C_1$ à $C_6$, alcoylthio en $C_1$ à $C_6$, alcoylsulfinyle en $C_1$ à $C_6$ ou alcoylsulfonyle en $C_1$ à $C_6$, ou
R et Z représentent ensemble

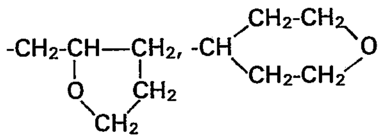 ou

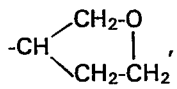 ,

$R_2$ représente un hydrogène, halogène, alcoyle en $C_1$ à $C_6$, alcoxy en $C_1$ à $C_6$, alcoylthio en $C_1$ à $C_6$ ou (alcoyle en $C_1$ à $C_6$)$_2$amino,
n représente les nombres 1 - 4, où $R_2$ peut être différent si n est l'un des nombres 2-4,
$R_3$ représente un halogène et
$R_6$ représente un alcoyle en $C_1$ à $C_6$, alcényle en $C_2$ à $C_6$ ou alcynyle en $C_2$ à $C_6$.

5. Composé selon la revendication 4, où
$R_1$ représente -A-R-Z,
A représente un oxygène ou un soufre,
$R_1$ représente un alcoylène en $C_2$ à $C_6$ non substitué,
Z représente un halogène, alcoxy en $C_1$ à $C_6$, alcoylthio en $C_1$ à $C_6$, alcoylsulfinyle en $C_1$ à $C_6$ ou alcoylsulfonyle en $C_1$ à $C_6$,
$R_2$ représente un hydrogène, halogène ou alcoyle en $C_1$ à $C_6$,
n représente le nombre 1,
$R_3$ représente un halogène et
$R_6$ représente un méthyle.

6. Composé selon la revendication 5, où
$R_1$ représente -A-R-Z en position 2 par rapport au groupe

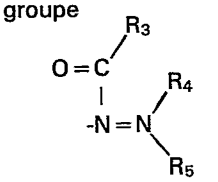 ,

A représente un oxygène,
R représente un alcoylène en $C_2$ à $C_6$ non substitué,
Z représente un halogène, alcoxy en $C_1$ à $C_4$, alcoylthio en $C_1$ à $C_4$, alcoylsulfinyle en $C_1$ à $C_4$ ou alcoylsulfonyle en $C_1$ à $C_4$,
$R_2$ représente un hydrogène, halogène ou alcoyle en $C_1$ à $C_4$,
n représente le nombre 1,
$R_3$ représente un halogène et
$R_6$ représente un méthyle.

7. Composé selon les revendications 2, 4, 5 et 6, où $R_3$ représente un fluor.

8. Composé selon la revendication 3 où
$R_1$ représente -A-R-Z,

A représente un oxygène, soufre, 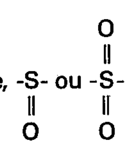

R représente un alcoylène en $C_2$ à $C_6$,
Z représente un halogène, halogènealcoyle en $C_1$ à $C_6$, alcoxy en $C_1$ à $C_6$, alcoylthio en $C_1$ à $C_6$, alcoylsulfinyle en $C_1$ à $C_6$ ou alcoylsulfonyle en $C_1$ à $C_6$ ou
R et Z représentent ensemble

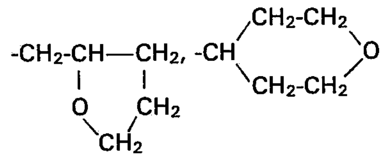 ou

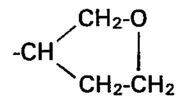 ,

$R_2$ représente un hydrogène, halogène, alcoyle en $C_1$ à $C_6$, alcoxy en $C_1$ à $C_6$, alcoylthio en $C_1$ à $C_6$ ou (alcoyle en $C_1$ à $C_6$)$_2$amino,
n représente les nombres 1 à 4, où $R_2$ peut être différent si n est l'un des nombres 2-4, et
$R_6$ représente un alcoyle en $c_1$ à $C_6$, alcényle en $C_2$ à $C_6$ ou alcynyle en $C_2$ à $C_6$.

9. Composé selon la revendication 8, où

A représente un oxygène, soufre, 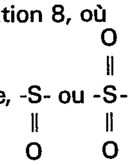

R représente un alcoylène en $C_2$ à $C_6$,
Z représente un halogène, alcoxy en $C_1$ à $C_6$, alcoylthio en $C_1$ à $C_6$, alcoylsulfinyle en $C_1$ à $C_6$ ou alcoylsulfonyle en $C_1$ à $C_6$,
$R_2$ représente un hydrogène, halogène, alcoxy en $C_1$ à $C_6$, alcoylthio en $C_1$ à $C_6$, ou (alcoyle en $C_1$ à $C_6$)$_2$amino,
n représente les nombres 1 à 4, où $R_2$ peut être différent si n est l'un des nombres 2-4, et
$R_6$ représente un alcoyle en $C_1$ à $C_6$, alcényle en $C_2$ à $C_6$ ou alcynyle en $C_2$ à $C_6$.

10. Composé selon la revendication 9, où
$R_1$ représente -A-R-Z,
A représente un oxygène ou un soufre,
R un alcoylène en $C_2$ à $C_6$,
Z un halogène, un alcoxy en $C_1$ à $C_6$, alcoylthio en $C_1$ à $C_6$, alcoylsulfinyle en $C_1$ à $C_6$ ou alcoylsulfonyle en $C_1$ à $C_6$,
n représente le nombre 1 et
$R_6$ représente un méthyle.

11. Composé selon la revendication 10, où
$R_1$ représente -A-R-Z en position 2 par rapport au groupe

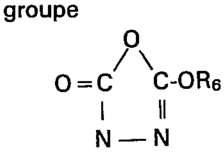 ,

A représente un oxygène,
R représente un alcoylène en $C_2$ à $C_4$ non substitué,
Z représente un halogène, alcoxy en $C_1$ à $C_4$, alcoylthio en $C_1$ à $C_4$, alcoylsulfinyle en $C_1$ à $C_4$ ou alcoylsulfonyle en $C_1$ à $C_4$,
$R_2$ représente un hydrogène, halogène ou alcoyle en $C_1$ à $C_4$,
n représente le nombre 1 et
$R_6$ représente un méthyle.

12. Le composé selon la revendication 6 de formule

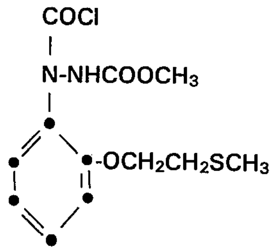

13. Le composé selon la revendication 6 de formule

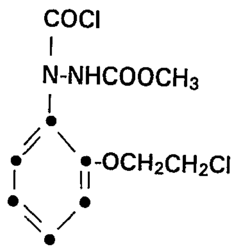

14. Le composé selon la revendication 6 de formule

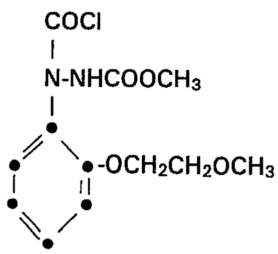

15. Le composé selon la revendication 5 de formule

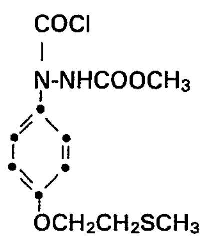

16. Le composé selon la revendication 6 de formule

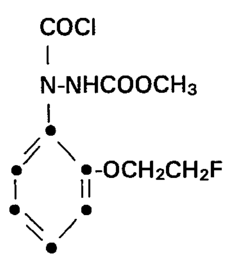

17. Le composé selon la revendication 6 de formule

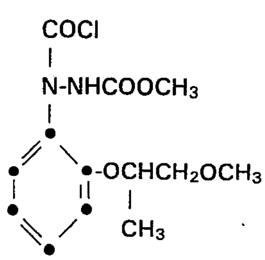

18. Le composé selon la revendication 6 de formule

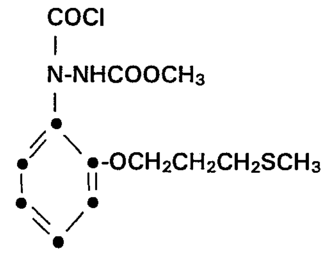

19. Le composé selon la revendication 6 de formule

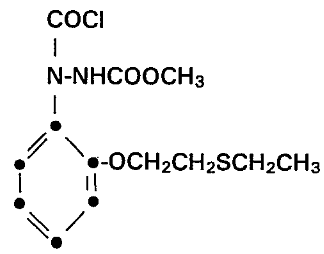

20. Le composé selon la revendication 6 de formule

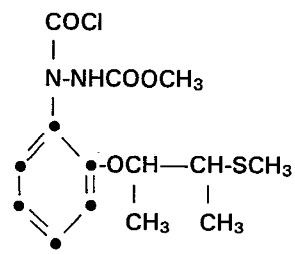

21. Le composé selon la revendication 6 de formule

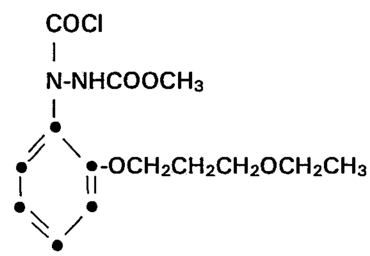

22. Le composé selon la revendication 6 de formule

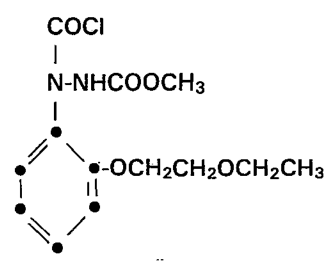

23. Le composé selon la revendication 4 de formule

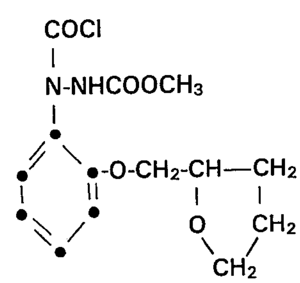

24. Le composé selon la revendication 4 de formule

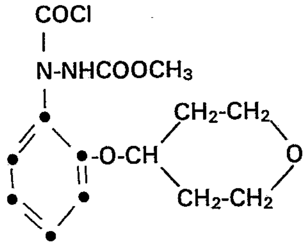

25. Le composé selon la revendication 7 de formule

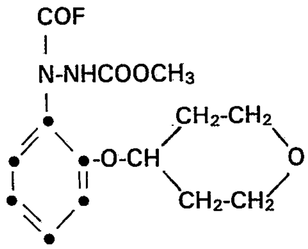

26. Le composé selon la revendication 6 de formule

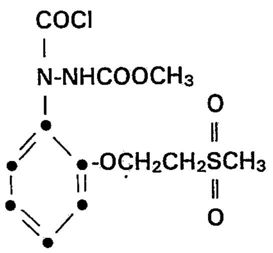

27. Le composé selon la revendication 6 de formule

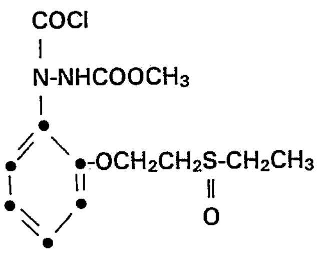

28. Le composé selon la revendication 6 de formule

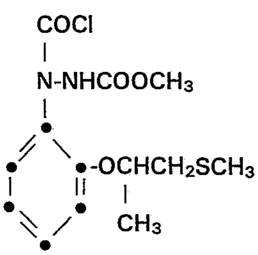

29. Le composé selon la revendication 7 de formule

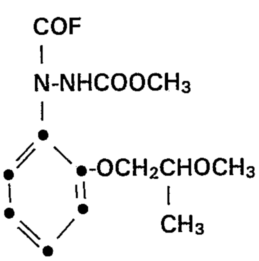

30. Le composé selon la revendication 7 de formule

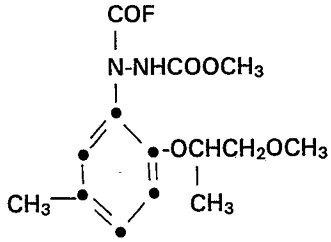

31. Le composé selon la revendication 7 de formule

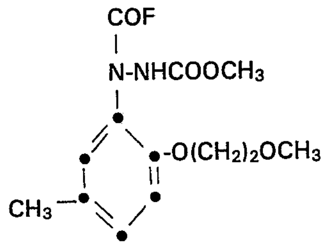

32. Le composé selon la revendication 7 de formule

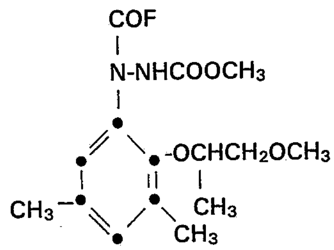

33. Le composé selon la revendication 7 de formule

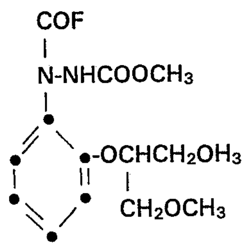

34. Le composé selon la revendication 7 de formule

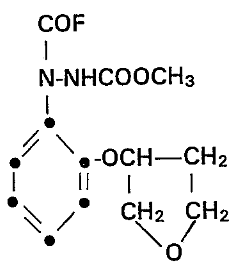

35. Le composé selon la revendication 11 de formule

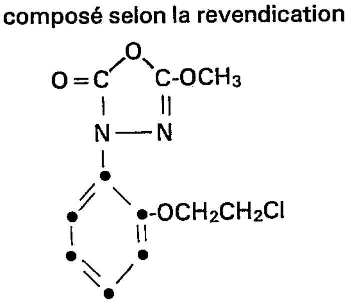

36. Le composé selon la revendication 11 de formule

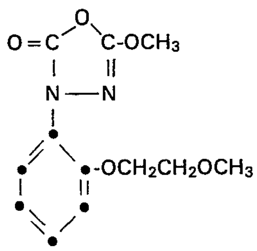

37. Le composé selon la revendication 11 de formule

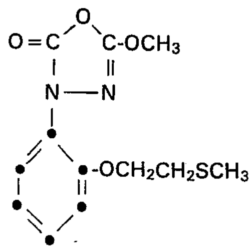

38. Le composé selon la revendication 11 de formule

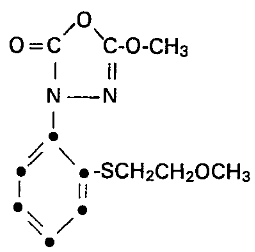

39. Le composé selon la revendication 11 de formule

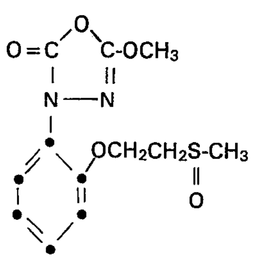

40. Le composé selon la revendication 11 de formule

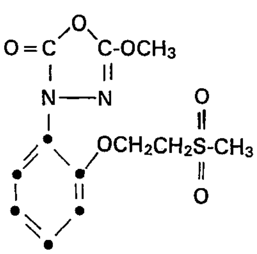

41. Le composé selon la revendication 11 de formule

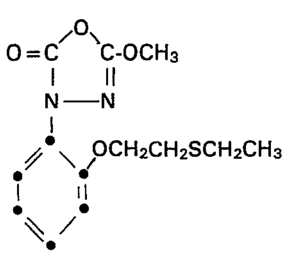

42. Le composé selon la revendication 11 de formule

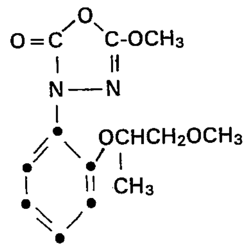

43. Le composé selon la revendication 11 de formule

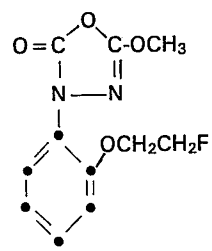

44. Le composé selon la revendication 11 de formule

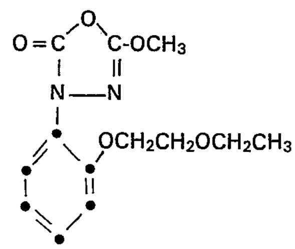

45. Le composé selon la revendication 8 de formule

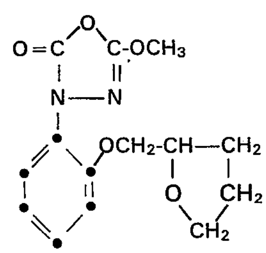

46. Le composé selon la revendication 11 de formule

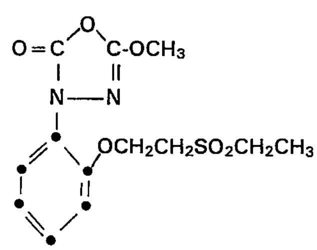

47. Le composé selon la revendication 8 de formule

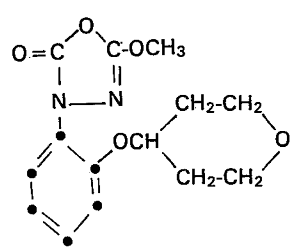

48. Le composé selon la revendication 11 de formule

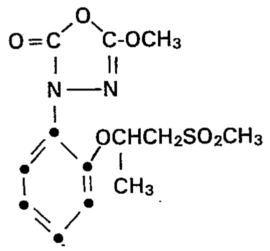

49. Le composé selon la revendication 11 de formule

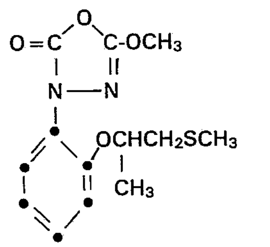

50. Le composé selon la revendication 3 de formule

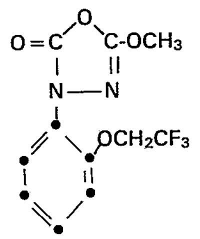

51. Le composé selon la revendication 11 de formule

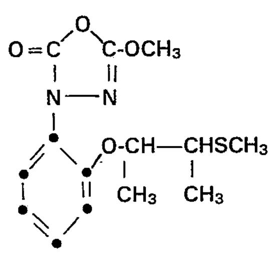

52. Le composé selon la revendication 10 de formule

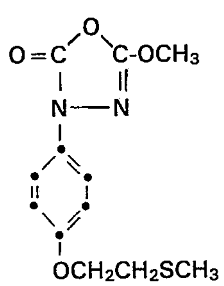

53. Le composé selon la revendication 11 de formule

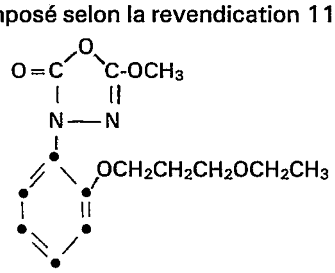

54. Le composé selon la revendication 3 de formule

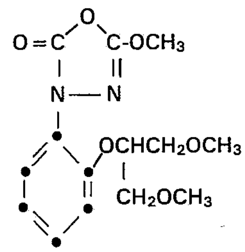

55. Procédé de préparation de composés de formule I selon la revendication 1, caractérisé en ce qu'on fait réagir un composé de formule II

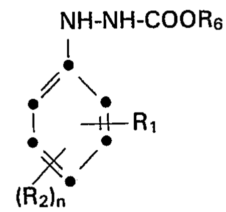

(II)

où $R_1$, $R_2$, $R_6$ et n ont la signification donnée pour la formule I, dans une première étape du procédé avec un composé de formule III

$CO(R_3)_2$ (III)

où $R_3$ représente un halogène puis en ce qu'on effectue une réaction de cyclisation sur le composé apparu de formule IV

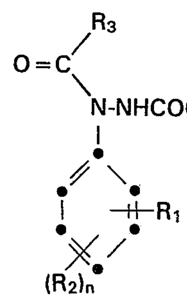

(IV)

où $R_1$, $R_2$, $R_3$, $R_6$ et n ont la signification donnée pour la formule I en présence d'une base.

56. Agent de lutte antiparasitaire, caractérisé en ce qu'il contient outre des supports et/ou autres additifs appropriés comme composant actif un composé selon la revendication 1.

57. Application de composés selon la revendication 1, à la lutte contre différentes espèces de parasites sur les animaux et les plantes ainsi que dans le sol.

58. Application selon la revendication 57 à la lutte contre les insectes et les représentants de l'ordre des acariens.

59. Procédé de lutte contre les parasites sur les animaux et les plantes ainsi que dans le sol, caractérisé en ce qu'on applique un composé selon la revendication 1 sur les animaux, les plantes ou le sol.